(19) Europäisches Patentamt
European Patent Office
Office européen des brevets

(11) Veröffentlichungsnummer: **0 471 303 A1**

(12) **EUROPÄISCHE PATENTANMELDUNG**

(21) Anmeldenummer: **91113377.5**

(22) Anmeldetag: **09.08.91**

(51) Int. Cl.5: **C07D 217/24**, C07D 217/20, C07D 491/056, C07D 498/04

(30) Priorität: **17.08.90 DE 4026115**

(43) Veröffentlichungstag der Anmeldung: **19.02.92 Patentblatt 92/08**

(84) Benannte Vertragsstaaten: **AT BE CH DE ES FR GB IT LI NL**

(71) Anmelder: **Consortium für elektrochemische Industrie GmbH**
**Zielstattstrasse 20**
**W-8000 München 70(DE)**

(72) Erfinder: **Hirsenkorn, Rolf, Dr.**
**Pullacher Strasse 23**
**W-8023 Pullach(DE)**
Erfinder: **Orlitsch, Silvia**
**Ottobrunner Strasse 11**
**W-8025 Unterhaching(DE)**

(54) **Verfahren zur Herstellung von 1-Alkylisochinolinderivaten.**

(57) Gegenstand der Erfindung ist ein Verfahren zur Herstellung von 1-Alkyl-1,2,3,4-tetra-hydroisochinolinderivaten der allgemeinen Formel

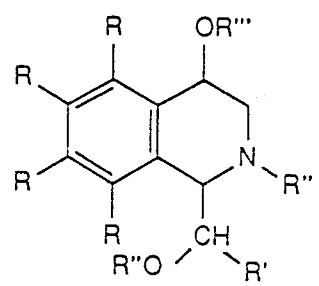

(1)

worin

- R = H- oder Halogenatome, Alkylamino-, Dialkylamino-, Acylamino-, Hydroxy-, Alkoxy-, Aralkoxy- oder Acyloxyreste oder jeweils 2 der Reste R zusammen einen Alkylendioxyrest bilden;
- R' = H-Atom oder Alkyl-, Acyl-, Aryl- oder mit Resten R substituierter Arylrest
- R'' = H-Atome, Acyl-, Alkyl-, Aryl- oder Silylreste oder 2 der Reste R'' zusammen einen Diacylrest bilden;
- R''' = H-Atome oder Alkylreste,

bei dem ausgehend von Verbindungen der allgemeinen Formeln

EP 0 471 303 A1

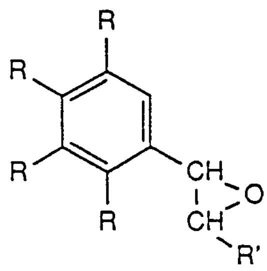 (2) oder 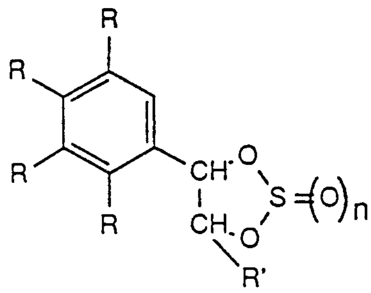 (3)

und Aminogruppen enthaltenden Verbindungen (4) über 2-Phenyl-2-aminoethanol-Verbindungen (5) der Ringschluß unter Bildung der 1-Alkyl-1,2,3,4-tetrahydro-isochinolin-derivate (1) über eine am Stickstoff befindliche Acetaldehyd- oder Acetaldehyddiacetalgruppe bei Temperaturen im Bereich von -20 ° C bis +50 ° C in Gegenwart von Säuren durchgeführt wird.

Die Verbindungen können für die Herstellung von Tetrahydro-isochinolinen vom Reticulintyp oder vom Norreticulintyp, oder für die Herstellung von Isochinolinen vom Papaverintyp verwendet werden.

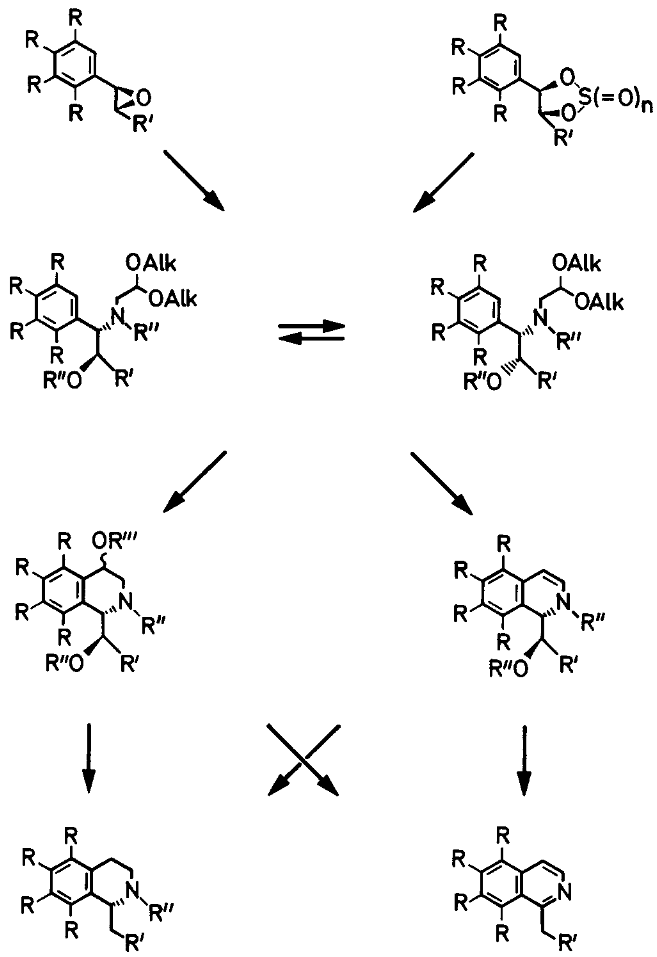

RETICULIN
NORRETICULIN
LAUDANOSIN
LAUDANOSOLIN
CARNEGIN
...ETC.

PAPAVERIN
...ETC.

Gegenstand der Erfindung ist ein Verfahren zur Herstellung von 1-Alkylisochinolinderivaten, worunter insbesondere 1-Alkyl-1,2,3,4-tetrahydro-isochinoline und 1-Alkyl-1,2-dihydro-isochinoline zu verstehen sind, in welchen die in 1-Stellung angeordneten Alkylreste auch substituiert sein können.

Das 1-Alkylisochinolingerüst ist der Grundbaustein für verschiedene pharmazeutische Wirkstoffe, beispielsweise für Antihypertensiva vom Salsolin-Typ, für Spasmolytica vom Papaverin-Typ oder für Analgetica vom Morphin-Typ. Derivate des 1-Alkylisochinolins sind daher wertvolle Zwischenprodukte für die Herstellung derartiger Wirkstoffe.

Für die Herstellung von 1-Alkylisochinolinen sind mehrere Verfahren bekannt, bei welchen entweder ausgehend von einem β-Phenylethylamin und anschließender Acylierung der Aminogruppe der Ringschluß zum Isochinolin in Gegenwart von Säuren bei erhöhter Temperatur vorgenommen wird (unter der Bezeichnung Bischler-Napieralski-Cyclisierung literaturbekannt); dabei entstehen 3,4-Dihydro-isochinoline.

Bei der Kondensation von β-Phenylethylaminen mit Carbonylverbindungen in saurer Lösung erfolgt hingegen der Ringschluß zum Isochinolin durch intramolekulare Aminomethylierung (unter der Bezeichnung Pictet-Spengler-Cyclisierung literaturbekannt); dabei entstehen 1,2,3,4-Tetrahydro-isochinoline.

Ein dritter Weg zur Herstellung von Isochinolinderivaten, bei dem aromatische Aldehyde mit Aminoacetalen kondensiert und die so erhältlichen Iminoacetale anschließend durch Säureeinwirkung cyclisiert werden (unter der Bezeichnung Pomeranz-Fritsch-Cyclisierung literaturbekannt) ist für die Herstellung von 1-substituierten, wie insbesondere 1-Benzylisochinolinderivaten praktisch nicht gangbar, da die entsprechenden aromatischen Ketone nicht nur schwer zugänglich sind, sondern diese auch mit Aminoacetalen nur schwierig oder gar nicht reagieren. Darüber hinaus erfolgt die anschließende Cyclisierung von Benzylaminoacetalen unter sauren Bedingungen praktisch ausschließlich unter Bildung des unerwünschten Isopavin- bzw. Pavingerüstes. (vgl. S. F. Dyke et al. in "Tetrahedron" Bd. 22, S. 3803 ff. (1971)). Die Cyclisierung von Benzylaminoacetalen ist präparativ erst nach Acylierung am Stickstoff durchführbar; dabei entstehen 1,2-Dihydro-isochinoline (vgl. K. Yamada et al. in "Chem. Pharm. Bull." Bd. 30, S. 3197 ff. (1982)).

Zur Vermeidung der Nachteile bei der Herstellung von in 1-Stellung substituierten Isochinolinderivaten nach Pomeranz-Fritsch, können zuerst die entsprechenden in 1-Stellung unsubstituierten Isochinolinderivate hergestellt und aus diesen durch Umsetzung mit Benzoylchlorid in Gegenwart von Kaliumcyanid 1-Cyano-2-benzoyl-1,2-dihydro-isochinoline hergestellt werden. Durch Reaktion mit Benzylchloriden und Hydrolyse unter alkalischen Bedingungen (unter der Bezeichung Reissert-Synthese literaturbekannt) können daraus die entsprechenden 1-Benzylisochinolinderivate erhalten werden (vgl. G. Blaskó et al. in "The Alkaloids", Bd. 31, S. 1 ff. Academic Press Inc. 1987)).

Nach den genannten Verfahren fallen die gewünschten 1-Alkylisochinolinderivate im allgemeinen in Form von Racematen an. Enantioselektive Synthesen sind jedoch ebenfalls bekannt. So wurde beispielsweise eine asymmetrische Totalsynthese von (+)-Reticulin beschrieben, die von einem in 1-Stellung unsubstituierten 1,2,3,4-Tetrahydro-isochinolinderivat ausgeht, das durch eine der oben beschriebenen Reaktionen zugänglich ist. Der Schlüsselschritt dieser Synthese ist die Deprotonierung des 1,2,3,4-Tetrahydro-isochinolins mit tert.-Butyllithium hei -78 °C und anschließende enantioselektive Alkylierung des 1-Lithio-1,2,3,4-tetrahydro-isochinolinderivats bei -100 °C mit einem entsprechend substituierten Benzylbromid. Die asymmetrische Induktion wird dabei von einem chiralen Formamidin ausgeübt. Nach dieser 5-stufigen Synthese wurde das (+)-Reticulin, ausgehend vom entsprechend substituierten Phenylethylamin, in 98,6 % ee und in einer Gesamtausbeute von ca. 30 % erhalten (vgl. A.I. Meyers et al. in "Heterocycles", Bd. 28, S. 295 ff. (1989)).

Diese bekannten Isochinolinsynthesen sind im allgemeinen vielstufig, und daher bei der Durchführung in technischem Maßstab unwirtschaftlich. Insbesondere die Pomeranz-Fritsch-Synthese, nach welcher in 1-Stellung unsubstituerte Isochinoline relativ einfach zugänglich sind, erfordert für die Synthese von 1-Benzyl-1,2,3,4-tetrahydro-isochinolinen zusätzliche Maßnahmen und ist daher besonders aufwendig und zeitraubend. Darüber hinaus erfordert die Einführung enantioselektiver Syntheseschritte beträchtlichen Aufwand.

Es stellt sich somit die Aufgabe, ein Verfahren zur Herstellung von in 1-Stellung substituierten Isochinolinderivaten zur Verfügung zu stellen, das von leicht zugänglichen Ausgangsprodukten ausgeht und das in wirtschaftlicher Weise die Herstellung der gewünschten wertvollen Zwischenprodukte in wenigen Stufen ermöglicht.

Das erfindungsgemäße Verfahren zur Herstellung von 1-Alkyl-1,2,3,4-tetra-hydro-isochinolinderivaten der allgemeinen Formel

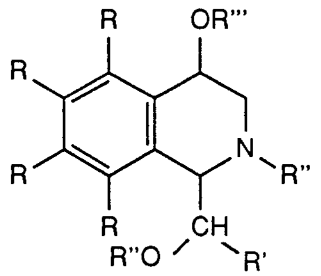

(1)

worin

R = H- oder Halogenatome, Alkylamino-, Dialkylamino-, Acylamino-, Hydroxy-, Alkoxy-, Aralkoxy- oder Acyloxyreste oder jeweils 2 der Reste R zusammen einen Alkylendioxyrest bilden;

R' = H-Atom oder Alkyl-, Acyl-, Aryl- oder mit Resten R substituierter Arylrest

R'' = H-Atome, Acyl-, Alkyl-, Aryl- oder Silylreste oder 2 der Reste R'' zusammen einen Diacylrest bilden;

R''' = H-Atome oder Alkylreste

ist dadurch gekennzeichnet, daß

(a) Verbindungen der allgemeinen Formeln

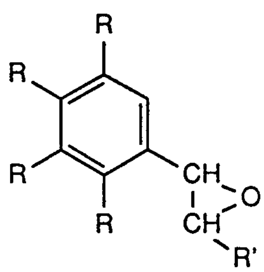

(2) oder

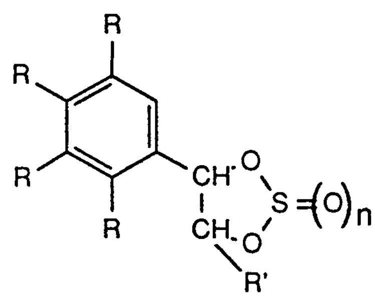

(3)

worin R und R' die angegebene Bedeutung haben und n = 1 oder 2, mit einerAminogruppen enthaltenden Verbindung (4) unter Ringöffnung bei Temperaturen im Bereich von 50 °C bis 200 °C in Gegenwart von protischen Lösungsmitteln oder Lewis-Säuren umgesetzt,

(b) in den so erhaltenen 2-Phenyl-2-aminoethanol-Verbindungen (5) freie OH-Gruppen und NH-Gruppen allein oder jeweils zusammen durch Schutzgruppen, ausgewählt aus Alkyl-, Acyl-, Silyl- und Diacylresten geschützt und

(c) der Ringschluß unter Bildung der 1-Alkyl-1,2,3,4-tetrahydro-isochinolinderivate (1) über eine am Stickstoff befindliche Acetaldehyd- oder Acetaldehyddiacetalgruppe bei Temperaturen im Bereich von -30 °C bis +40 °C in Gegenwart von Säuren durchgeführt wird.

Die bei dem erfindungsgemäßen Verfahren in der ersten Verfahrensstufe (a) als Ausgangsverbindungen eingesetzten Verbindungen (2), worunter Derivate des Styroloxids zu verstehen sind, können in an sich bekannter Weise durch Oxidation von entsprechend substituierten Styrolen hergestellt werden. Beispiele für Oxidationsmittel sind Natriumhypochlorit und m-Chlorperoxybenzoesäure.

Es ist jedoch auch möglich, optisch aktive Styroloxidderivate herzustellen, beispielsweise durch enantioselektive, katalytische Epoxidation an chiralen Mangan-Salen-Komplexen (vgl. Wei Zhang, et al. in "J. Am. Chem. Soc.", Bd. 112, S. 2801 ff. (1990)). Auf diese Weise ist es möglich optisch aktives Ausgangsmaterial zu verwenden, so daß bei der anschließenden Durchführung des erfindungsgemäßen Verfahrens optisch aktive Verbindungen (1), welche also Enantiomerenverhältnisse von ungleich 50 : 50 besitzen, erhalten werden.

Unter den Ausgangsverbindungen (3) sind Analoga der Styroloxidderivate zu verstehen, die anstelle des Oxidsauerstoffes eine cyclische Sulfit- oder Sulfatestergruppierung aufweisen. Zu ihrer Herstellung können in an sich bekannter Weise entsprechend substituierte Styrole mit ausgewählten Oxidationsmitteln (z. B. N-Methylmorpholin) an chiralen Osmium-Katalysatoren enantioselektiv dihydroxyliert werden (vgl. E.N. Jacobsen et al. in "J. Am. Chem. Soc.", Bd. 110, S. 1968 ff. (1988)).

Die so erhaltenen Phenyl- 1,2-ethandiole können beispielsweise durch Einwirkung von Thionylchlorid unter Bildung der Sulfitester verestert werden und diese anschließend unter Bildung der Sulfatester oxidiert

werden, beispielsweise unter Verwendung von Natriumperjodat als Oxidationsmittel in Gegenwart von Ruthenium (III)-chlorid (vgl. B. M. Kim et al. in "Tetrahedron Letters", Bd. 30, S. 655 ff. (1989)).

Auch in diesem Falle geht die Synthese von optisch aktivem Ausgangsmaterial aus, so daß bei der anschließenden Durchführung des erfindungsgemäßen Verfahrens optisch aktive Verbindungen (1) erhalten werden.

Die Ringöffnung von substituierten Ausgangsverbindungen (2) oder (3) (R' ungleich H-Atom) durch das Amin (4) erfolgt stereoselektiv, d. h. je nach Konfiguration der Ausgangsverbindung werden die entsprechenden Diastereomeren 2-Phenyl-2-aminoethanolverbindungen (5) erhalten. Beispielsweise werden aus trans-konfigurierten Verbindungen (2) oder (3) die erythro-Derivate, aus cis-konfigurierten Verbindungen (2) oder (3) hingegen die threo-Derivate erhalten. Diese Diastereomeren können nach Bedarf nach an sich bekannten Verfahren, beispielsweise durch Erhitzen in einem Gemisch Trifluoressigsäure / Trifluoressigsäureanhydrid ineinander umgewandelt werden (vgl. D. Seebach et al. in "Helv. Chim. Acta, Bd. 70, 1357 ff. (1987)).

In den Ausgangsverbindungen (2) und (3) haben die Reste R und R' praktisch die gleiche Bedeutung wie in den gewünschten Isochinolinderivaten (1).

Beispiele für derartige Reste R, die jeweils gleich oder verschieden sein können, sind außer H-Atomen und Hydroxylgruppen, Halogenatome, wie Fluor-, Chlor-, Brom- und Jodatome; Alkoxyreste mit 1-5 C-Atomen, die geradkettig oder verzweigt sein können, wie vorzugsweise Methoxy- und Ethoxyreste; Aralkoxyreste, wie der Benzyloxyrest; Alkylendioxyreste, wie Methylendioxy- und Ethylendioxyreste, sowie Acyloxyreste, wie Acetoxy-, Trichloracetoxy- und Benzoyloxyreste.

Beispiele für derartige Reste R' sind außer H-Atomen, Alkylreste mit 1-5 C-Atomen, die geradkettig oder verzweigt sein können; Acylreste, wie Formyl-, Acetyl-, Propionyl-, Benzoyl-, Carboxymethyl-, Carboxyethyl- und Carboxybenzylreste; Arylreste wie Phenyl oder mit Resten R substituierte Phenylreste.

Bei der Durchführung des erfindungsgemäßen Verfahrens werden die Verbindungen (2) oder (3) in der Verfahrensstufe (a) definitionsgemäß mit der Aminogruppen enthaltenden Verbindung (4) unter Öffnung des Epoxidringes der Verbindung (2) oder der cyclischen Sulfit- bzw. Sulfatestergruppierung der Verbindung (3) umgesetzt.

Als Aminogruppen enthaltende Verbindungen (4) können Ammoniak und primäre oder sekundäre Amine verwendet werden. Die Umsetzung wird definitionsgemäß entweder in Gegenwart von protischen Lösungsmitteln oder unter wasserfreien Bedingungen in Gegenwart von Lewis-Säuren durchgeführt. Die Verbindungen (2) bzw. (3) und (4) werden dabei im Molverhältnis von etwa 1 : 20 bis 4 : 5 eingesetzt, d. h. es wird mit einem Aminüberschuß gearbeitet.

Beispiele für protische Lösungsmittel sind Alkohole, wie Methanol, Ethanol, iso-Propanol, n-Propanol, sec. Butanol und tert. Butanol oder das eingesetzte Amin (4). Bevorzugt werden Ethanol und iso-Propanol als protische Lösungsmittel eingesetzt.

Zusätzlich können inerte Lösungsmittel mitverwendet werden, welchen etwa 5 bis 50 Vol.-%, vorzugsweise etwa 10 Vol.-% eines protischen Lösungsmittels zugesetzt werden.

Beispiele für inerte Lösungsmittel sind Kohlenwasserstoffe wie Benzol und Toluol; halogenierte Kohlenwasserstoffe, wie Chlorbenzol, Chloroform, Dichlormethan und Perchlorethylen oder Ether wie Diethylether, tert. Butylmethylether, Tetrahydrofuran und Dioxan, wobei Chloroform und Dichlormethan bevorzugt sind.

Die Temperatur für die Umsetzung ist abhängig von den verwendeten Ausgangsverbindungen und den ausgewählten Lösungsmitteln. Vorzugsweise wird bei Temperaturen im Bereich von etwa 70°C bis 170°C gearbeitet. Die Lösungsmittel können im Verlauf der Reaktion aus dem Reaktionsgemisch abdestilliert werden, wobei es in diesem Fall vorteilhaft ist, wenn das inerte Lösungsmittel einen tieferen Siedepunkt als das protische Lösungsmittel hat.

Bei Einsatz von niedrigsiedenden Aminen, wie Ammoniak oder Methylamin wird die Reaktion vorteilhaft in einem Druckgefäß durchgeführt.

Die Reaktion kann durch Zusätze wie Tetraalkylammoniumhalogenid, Aluminiumoxid oder Kieselgel katalysiert werden, dies ist für Epoxidöffnungen allgemein bekannt. Die Reaktionstemperatur liegt dann tiefer.

Anstelle der protischen Lösungsmittel können Lewis-Säuren verwendet werden, die vorteilhaft im stöchiometrischen Verhältnis zu den Verbindungen (2) bzw. (3) eingesetzt werden. Beispiele hierfür sind Zink(II)chlorid, Bortrifluorid, Aluminium(III)isopropylat und Titan(IV)isopropylat. Die Umsetzung wird vorteilhaft in einem der o.g. inerten Lösungsmittel unter wasserfreien Bedingungen durchgeführt; die Reaktionstemperatur liegt hierbei ebenfalls tiefer, d.h. vorzugsweise bei Raumtemperatur.

Der Einsatz der Reaktionspartner im Molverhältnis Amin : Epoxid von 2 : 1, sowie der Zusatz von etwa 10 Gew.-% Kieselgel (bezogen auf eingesetztes Epoxid) im Lösungsmittelgemisch Dichlormethan/iso-Propanol im Volumenverhältnis 9 : 1 unter Schutzgasatmosphäre, anschließender langsamer Erwärmung auf

20°C bis 150°C unter gleichzeitigem Abdestillieren des Lösungsmittelgemisches und Abkühlen auf 20°C nach beendeter Umsetzung hat sich für die Durchführung der Verfahrensstufe a) besonders bewährt. Das verbleibende Reaktionsgemisch wird dann nach hydrolytischer Aufarbeitung mit einem Lösungsmittel extrahiert und eingedampft.

Wenn in der Verfahrensstufe a) die Ringöffnung der Verbindungen (2) bzw. (3) mit Ammoniak oder einem primären Alkylamin, wie Methylamin durchgeführt worden ist, werden als Verbindungen (5) 2-Phenyl-2-aminoethanole oder 2-Phenyl-2-alkylaminoethanole erhalten.

Um die für den Isochinolinringschluß in der Verfahrensstufe (c) erforderliche, am N-Atom befindliche Acetaldehyd- oder Acetaldehyddiacetalgruppierung einzuführen, müssen diese Verbindungen mit beispielsweise Halogenacetaldehyden, wie Chlor- oder Bromacetaldehyd oder Halogenacetaldehyddialkylacetalen, wie Chlor- oder Bromacetaldehyddimethylacetal in an sich bekannter Weise in Gegenwart einer Base umgesetzt werden.

Beispiele für Basen sind anorganische Basen, wie Natriumhydrid, Natriumcarbonat, Natriumhydrogencarbonat oder organische Basen wie Triethylamin, Pyridin und Dimethylaminopyridin.

Die Einführung der Acetaldehydgruppierung kann auch durch Umsetzung mit Ethylenoxid und anschließender Oxidation des primären Alkohols zum Acetaldehyd-Derivat erfolgen.

Die Umsetzung mit Ethylenoxid kann bei Temperaturen von etwa 70° bis 130°C in einem Druckgefäß in Gegenwart eines Lösungsmittels vorgenommen werden. Als Lösungsmittel und Katalysatoren können die gleichen, wie die in der Verfahrensstufe (a) genannten verwendet werden.

Die Oxidation zum Acetaldehyd-Derivat erfolgt in an sich bekannter Weise mit Dimethylsulfoxid/Oxalylchlorid.

Vorzugsweise wird die Ringöffnung der Verbindungen (2) bzw. (3) in der Verfahrensstufe (a) jedoch mit primären oder sekundären Aminen durchgeführt, die bereits die für den Isochinolinringschluß in der Verfahrensstufe (c) benötigte Acetaldehydgruppierung enthalten. Hierunter sind insbesondere Aminoacetaldehyddialkylacetale, wie Aminoacetaldehyddimethylacetal und Alkylaminoacetaldehyddialkylacetale wie Methylaminoacetaldehyddimethylacetal zu verstehen.

Bevor die Isochinolinringschlußreaktion gemäß Verfahrensstufe (c) in Angriff genommen werden kann, muß in der Verfahrensstufe (b) die freie OH-Gruppe in der 2-Aminoethanolgruppierung der Verbindungen (5) geschützt werden. Wenn die Verbindungen (5) sekundäre Amine sind, in welchen am N-Atom ein freies H-Atom vorhanden ist, kann die NH-Gruppe ebenfalls geschützt werden.

Die Einführung von Schutzgruppen kann in an sich bekannter Weise vorgenommen werden, beispielsweise durch Alkylierung mit Alkylhalogeniden, wie Methyl- und Ethyljodid oder Benzylchlorid; durch Acylierung mit Carbonsäurehalogeniden, wie Benzoylchlorid, Pivaloylchlorid, Pivaloylmandelsäurechlorid oder Trichloracetylchlorid; mit Carbonsäureestern, wie Chlorameisensäureester; mit Carbonsäureanhydriden, wie Acetanhydrid und Trifluoracetanhydrid. Derartige Reaktionen werden üblichersweise in Gegenwart einer Base wie Natriumcarbonat, Triethylamin und Pyridin durchgeführt. Bekannt ist ferner die Silylierung mit Hilfe von Alkoxy- oder Chlorsilanen. Bei Einsatz von Schutzgruppen liefernden Substanden mit zwei funktionellen Gruppen können auch OH- und NH-Gruppe gemeinsam unter Ringbildung geschützt werden, beispielsweise durch Umsetzung mit Oxalylchlorid, mit Dialkylcarbonaten, wie Dimethylcarbonat, Diethylcarbonat oder Ethylencarbonat oder mit N,N'-Carbonyl-diimidazol.

Nachdem die freien OH-Gruppen in der vorbeschriebenen Weise geschützt worden sind, kann die Isochinolinringschlußreaktion definitionsgemäß nach Verfahrensstufe (c) in Gegenwart von Säuren vorgenommen werden.

Als Säuren werden vorteilhaft wässrige Mineralsäuren, wie Schwefelsäure, Phosphorsäure und Salzsäure verwendet, wobei sich konzentrierte Salzsäure besonders bewährt hat.

Die Säuren werden zusammen mit wassermischbaren Lösungsmitteln im Volumenverhältnis von 10 : 90 bis 90 : 10 eingesetzt. Beispiele für mit Wasser mischbare Lösungsmittel sind Alkanole, wie Methanol, Ethanol, iso-Propanol, n-Propanol, sek. Butanol und tert. Butanol; Ether wie Tetrahydrofuran und Dioxan sowie Ketone wie Aceton und Butan-2-on.

Besonders bewährt hat sich ein Gemisch aus Aceton und konzentrierter Salzsäure im Volumenverhältnis von 40 : 60 bis 80 : 20.

Anstelle der wässrigen Mineralsäuren können jedoch auch Lewis-Säuren zusammen mit wasserfreien Lösungsmitteln verwendet werden. Beispiele für Lewis-Säuren sind Titantetrachlorid, Bortrifluorid, Bortrifluorid / Diethyletherkomplexverbindungen und Zinntetrachlorid, wobei sich Bortrifluorid / Diethyletherkomplexverbindungen und Titantetrachlorid besonders bewährt haben.

Beispiele für wasserfreie Lösungsmittel sind Kohlenwasserstoffe, wie Hexan, Petrolether und Benzol; Chlorkohlenwasserstoffe wie Dichlormethan, Chloroform, Dichlorethan und Perchlorethylen; Ether wie Diethylether, tert. Butylmethylether, Diethylenglykoldimethylether und Tetrahydrofuran. Als wasserfreie Lösungsmittel sind Dichlormethan und Diethylether bevorzugt.

Die Menge der Lewis-Säure kann im Bereich von katalytischen bis zu stöchiometrischen Mengen liegen.

Beide Verfahren, d. h. sowohl die Durchführung des Verfahrens mit wässrigen Mineralsäuren, als auch mit Lewis-Säuren unter wasserfreien Bedingungen sind durch folgende Reaktionparameter gekennzeichnet:

Anfangstemperatur von -20° bis +10° C, vorzugsweise etwa -5° C und Endtemperatur von +10° bis +50° C, vorzugsweise Raumtemperatur;

Druck: Normaldruck, bzw. Eigendruck.

Wenn im Reaktionsgemisch eine Temperatur von +15° C erreicht ist, wird die Reaktion beispielsweise dünnschichtchromatographisch kontrolliert und abgebrochen, wenn das Acetal bzw. der Aldehyd verbraucht ist. Die Reaktionszeit kann bei Raumtemperatur im allgemeinen etwa 0,5 bis 15 Stunden betragen, üblicherweise ist die Reaktion nach etwa 3 bis 5 Stunden beendet.

Die Verfahrensstufe (c) kann diskontinuierlich oder kontinuierlich durchgeführt werden, wobei eine kontinuierliche Verfahrensführung aufgrund der definierten Verweilzeit und Dosierung von Vorteil ist.

Besonders bewährt hat es sich für die Verfahrensstufe (c) wenn die Reaktionsteilnehmer bei tiefer Temperatur vereinigt und dann langsam auf Raumtemperatur erwärmt werden, bei welcher die eigentliche Cyclisierung stattfindet. Abschließend wird das Reaktionsgemisch alkalisch-hydrolytisch aufgearbeitet, extrahiert und das Lösungsmittel entfernt.

Bei der Verfahrensstufe (c) werden im allgemeinen Ausbeuten von mehr als 55 % erreicht, ohne daß nachweisbare Mengen an Verbindungen mit der unerwünschten Isopavin- oder Pavinstruktur gebildet werden.

Die so hergestellten Verbindungen der allgemeinen Formel (1) sind 1,2,3,4-Tetrahydro-isochinolinderivate, die in 1-Stellung des Isochinolinringes einen ggf. substituierten Hydroxalkyl-Rest enthalten und in 4-Stellung eine Hydroxy- oder Alkoxy-Gruppe tragen. Beide Hydroxylgruppen können entweder in freier Form vorliegen oder mit Schutzgruppen versehen sein.

Aus diesen Verbindungen (1), die wertvolle Zwischenprodukte sind, können in Nachfolgereaktionen die beiden Hydroxylgruppen oder die mit Schutzgruppen versehenen Hydroxylgruppen entweder gleichzeitig oder nacheinander entfernt werden.

Das kann beispielsweise durch Reduktion mit komplexen Hydriden vorgenommen werden, wobei durch Umsetzung mit einem Halogenierungsmittel in Gegenwart einer Base die entsprechenden Halogen-substituierten Isochinoline hergestellt und diese dann mit einem Hydrid-Donor zu der entsprechenden gesättigten Verbindung reduziert werden.

Beispiele für Halogenierungsmittel sind Thionylchlorid, Phosphortrichlorid, Phosphorpentachlorid, Phosphoroxychlorid, Phosphortribromid, Sulfonylchlorid und Bromwasserstoff, wobei Thionylchlorid bevorzugt ist. Beispiele für Basen sind tertiäre Amine, wie Triethylamin und Pyridin. Beispiele für Reduktionsmittel sind Hydrid-donatoren, wie Lithiumaluminiumhydrid, Natriumaluminiumhydrid, Natrium-bis-(methoxyethoxy)-aluminiumhydrid, Natriumborhydrid, wobei Lithiumaluminiumhydrid und Natriumborhydrid bevorzugt sind. Die Reaktion wird zweckmäßig unter wasserfreien Bedingungen in Gegenwart von inerten Lösungsmitteln durchgeführt, wobei Tetrahydrofuran bevorzugt ist. Je zu reduzierender OH-Gruppe werden 1 - 2 Equivalente Halogenierungsmittel, 1,1 - 2,2 Equivalente Base und 1,2 - 10 Equivalente Reduktionsmittel eingesetzt.

Allgemein ausgedrückt wird die Hydroxylverbindung (1) in einem inerten Lösungsmittel bei vorzugsweise 0° C mit dem Halogenierungsmittel versetzt und die in situ entstehende Halogenverbindung unmittelbar anschließend hei 0° C bis 20° C zu der gesättigten Verbindung reduziert. Nach hydrolytischer Aufarbeitung wird mit einem Lösungsmittel extrahiert und eingedampft.

In analoger Weise können aus den Benzylalkoholderivaten auch die entsprechenden Tosylate hergestellt und diese anschließend reduziert werden.

In analoger Weise können aus den Benzylalkoholderivaten auch die entsprechenden Acetate hergestellt und diese anschließend in an sich bekannter Weise z.B. mit Natriumborhydrid in Gegenwart von Nickelacetat reduziert werden (vgl. Yun He et al. in "Synthetic Communications", Bd. 19 S. 3051 ff (1989)).

Anstelle der Reduktion mit komplexen Hydriden kann jedoch auch eine katalytische Hydrierung durchgeführt werden. Zur Reduktion von 4-Hydroxy- oder 4-Alkoxy-Substituenten muß in Gegenwart einer Säure hydriert werden. Beispiele für $H_2$-Quellen sind molekularer Wasserstoff, der bevorzugt ist, aber auch Wasserstoffüberträger (Transferhydrierung), wie Ammoniumformiat, Ameisensäure / Triethylamin und Alkohole. Beispiele für Katalysatoren sind Edelmetalle wie Palladium und Platin oder Raney-Nickel als metallischer Niederschlag und Salze bzw. Komplexsalze und Oxide wie $PdCl_2$, PdO, $PtO_2$ auf üblichen Trägern, wie Aktivkohle; wobei sich ein Palladiumkatalysator mit etwa 10 % Pd auf Aktivkohle besonders bewährt hat. Beispiele für Säuren sind Mineralsäuren wie Salzsäure, Schwefelsäure oder Phosphorsäure oder

Carbonsäuren wie Ameisensäure, Essigsäure, Propionsäure, Oxalsäure, Malonsäure, Citronensäure, Apfelsäure, Salicylsäure, wobei Essigsäure und Oxalsäure bevorzugt sind. Als Lösungsmittel sind protische Lösungsmittel oder solche, die mit Wasser mischbar sind, bevorzugt. Beispiele für solche Lösungsmittel sind Tetrahydrofuran, Methanol, Ethanol, Isopropanol. Sie können als solche oder im Gemisch mit Wasser verwendet werden. Der molekulare Wasserstoff kann mit einem Druck im Bereich von ca. 0,1 - 15,0 MPa angewendet werden; die Temperatur liegt vorteilhaft im Bereich von 50 °C bis 100 °C.

Wenn in den Zwischenprodukten gemäß der allgemeinen Formel (1) ein tertiäres Stickstoffatom vorhanden ist, d. h. beispielsweise der Stickstoff mit Methylsubstituiert ist, werden durch diese reduktiven Maßnahmen 1,2,3,4-Tetrahydro-isochinoline vom Reticulintyp erhalten, die ihrerseits als Bausteine z.B. für Morphinsynthesen brauchbar sind. Beispiele für solche Verbindungen sind Reticulin, Laudanosin, Laudanosolin, Carnegin und 6-(Dimethylamino)-1-(3-hydroxy-4-methoxybenzyl)-1,2,3,4-tetrahydro-isochinolin. Bei Vorhandensein eines sekundären Stickstoffatoms, d. h. einer freien NH-Gruppe werden 1,2,3,4-Tetrahydroisochinoline vom Norreticulintyp erhalten. Beispiele für solche Verbindungen sind Norreticulin, Norlaudanosin, Salsolidin, Salsolin und Salsolinol.

Aus Verbindungen (1), die am Stickstoff eine Acyl-Schutzgruppe tragen, können ferner durch mit Säuren katalysierte Hydrolyse sowohl die Acyl- und andere Schutzgruppen abgespalten und gleichzeitig die Hydroxylgruppen entfernt werden, so daß auf diese Weise direkt Isochinoline vom Papaverintyp erhalten werden. Die Reaktion wird vorteilhaft in einem inerten Lösungsmittel wie Toluol oder Xylol durchgeführt, als Säure wird vorzugsweise p-Toluolsulfonsäure verwendet. Es wird bei erhöhter Temperatur, vorzugsweise bei der Rückflußtemperatur des Lösungsmittels gearbeitet.

Als Endprodukte werden hierbei aromatisierte Isochinoline, beispielsweise vom Papaverintyp erhalten.

Ferner ist es unter bestimmten Bedingungen möglich, daß in Abänderung des erfindungsgemäßen Verfahrens durch Zusammenfassung der Stufen (b) und (c) unter gleichzeitiger Entfernung der Hydroxylgruppe in 4-Stellung direkt 1,2-Dihydro-isochinolinderivate erhalten werden. So können beispielsweise durch eine kombinierte Acylierung und Cyclisierung von Verbindungen (5), die sekundäre Amine sind, d.h. in welchem am Stickstoffatom nach der Ringöffnung gemäß Verfahrensstufe (a) ein freies H-Atom vorhanden ist, wie Aminoacetaldehyddialkylacetale, in Gegenwart von Acylierungsmitteln, wie Carbonsäurehalogeniden, insbesondere Benzoylchlorid und in Gegenwart von Basen, wie tertiären Aminen und Pyridin unter wasserfreien Bedingungen 1,2-Dihydro-isochinolinderivate direkt in einem Verfahrensschritt erhalten werden, in welchen die OH-Gruppe in 4-Stellung bereits beim Ringschluß entfernt worden ist Durch anschließende weitere Reduktion zur Entfernung der N- und O-Acylgruppe mit beispielsweise Hydrazinhydrat können daraus direkt aromatische Isochinoline wie Papaverin gewonnen werden, entsprechende Substituenten vorausgesetzt. Aus entsprechend substituierten 1,2-Dihydro-isochinolinderivaten können jedoch auch durch Umsetzung mit sauerwirkenden Verbindungen, wie Mineralsäuren, vorzugsweise Salzsäure, in Gegenwart von mit Wasser mischbaren Lösungsmitteln, vorteilhaft bei Rückflußtemperatur, direkt aromatische Isochinolinderivate beispielsweise vom Papaverintyp erhalten werden.

In Abbildung 1 sind schematisch die Verfahrensschritte nach dem erfindungsgemäßen Verfahren zur Herstellung der Verbindungen (1) und mögliche Nachfolgereaktionen vereinfacht dargestellt.

## Beispiele:

### Herstellung der Ausgangsverbindungen:

### Beispiel 1: rel-(S,S)-3,3'-Dibenzyloxy-4,4'-dimethoxy-stilbenoxid

**Ansatz:**
66,7g (150mmol) 3,3'-Dibenzyloxy-4,4'-dimethoxy-stilben
1500ml Dichlormethan
1500ml Natriumhydrogencarbonat-Lösung
667mg (1,5mmol) Nickel-Salen
1,334g (4,3mmol) Benzyltributylammoniumchlorid
250ml Natriumhypochlorit-Lösung (13% aktives Chlor)

**Durchführung:** Bei 5 bis 10 °C wurde die Natriumhypochlorit-Lösung während 2h zum Gemisch der übrigen Reagenzien zugetropft und 2h nachgerührt. Zur Aufarbeitung wurde die organische Phase abgetrennt und über eine kurzes Kieselbett filtriert. Die so hergestellte Epoxidlösung steht für weitere Umsetzungen direkt zur Verfügung oder kann einrotiert und aus Ethanol umkristallisiert werden.

**Ausbeute:** 66,5g (96,4%)

**Anal.Daten:** $R_f$(Dichlormethan/Methanol 100:0) = 0,50 $R_f$(Edukt) = 0,90;
**Smp:** 102° (Ethanol)

**Beispiel 2: rel-(S,S)-3,3',4,4'-Tetramethoxy-stilbenoxid**

**Ansatz:** 50,0g (166mmol) 3,3'4,4'-Tetramethoxystilben
1000ml Dichlormethan
1000ml Natriumhydrogencarbonat-Lösung
64,0g (185mmol) m-Chlorperoxybenzoesäure (ca. 50%ig)

**Durchführung:** Zum Tetramethoxystilben in Dichlormethan und Natriumhydrogencarbonat wurde die m-Chlorperoxybenzoesäure, gelöst in 200ml Dichlormethan, langsam zugetropft (ca.4h). Nach 1h wurde die organische Phase abgetrennt. Die so hergestellte Epoxidlösung steht für weitere Umsetzungen zur Verfügung.

**Ausbeute:** die Umsetzung verlief quantitativ
**Anal.Daten:** $\mathbf{R_f}$(Dichlormethan) = 0,33; $\mathbf{R_f}$(Edukt) = 0,53

**Beispiel 3: 3,4-Dimethoxy-styroloxid**

**Ansatz:** 27,2g (160mmol) 3,4-Dimethoxystyrol
600ml Dichlormethan
220ml Natriumhydrogencarbonat-Lösung
7,1g Benzyltributylammoniumchlorid
1,15g (3,5mmol) Nickel-Salen
900ml 13%ige Natriumhypochlorit-Lösung

**Durchführung:** Dimethoxystyrol, Benzyltributylammoniumchlorid und Nickel-Salen-Katalysator wurden in Dichlormethan und Natriumhydrogencarbonat vorgelegt, die Natriumhypochlorit-Lösung wurde während 4h zugetropft. Danach wurde 1h gerührt, die organische Phase abgetrennt, mit gesättigter Natriumchlorid-Lösung gewaschen und über Kieselgel filtriert. Die so hergestellte Epoxidlösung wurde direkt für weitere Umsetzungen verwendet.

**Ausbeute:** die Umsetzung verlief quantitativ
**Anal.Daten:** $\mathbf{R_f}$(Dichlormethan) = 0,33; $\mathbf{R_f}$(Edukt) = 0,62

**Beispiel 4: (1S,2S)-1,2-Bis-(3'-benzyloxy-4'-methoxyphenyl)-ethandiol**

**Ansatz:** 50g (110mmol) 3,3'-Dibenzyloxy-4,4'-dimethoxystilben
0,49g (1,66mmol) Osmiumtrichlorid
2,57g (5,33mmol) 4-Chlorbenzoyl-dihydrochinidin
22,4g (191mmol) N-Methylmorpholin-N-oxid
31 Aceton/Wasser (9:1)
30g Kieselgel

**Durchführung:** Das Stilben wurde, vermengt mit dem Kieselgel, zum Gemisch der übrigen Reagenzien bei Raumtemperatur während 48h möglichst kontinuierlich zudosiert. Zur Aufarbeitung wurde vom Kieselgel abfiltriert, die Lösung eingeengt und der Rückstand mit 400ml Tetrahydrofuran/gesätt. Natriumbisulfit-Lösung (1:1) 2h unter Rückfluß erhitzt. Die organische Phase wurde abgetrennt, die wässrige Phase wurde mit gesätt. Natriumhydrogencarbonat-Lösung auf pH = 9 gebracht und mit Ethylacetat extrahiert. Die vereinten organischen Phasen wurden eingeengt und chromatographiert (Dichlormethan/Methanol 95:5).

**Ausbeute**: 47,3g (88,4%)
**Anal.Daten:** $\mathbf{R_f}$(Dichlormethan/Methanol 95:5) = 0,38; $\mathbf{R_f}$(Edukt) = 0,95
$[\alpha]_D$ = +120,3° (c = 1, Dichlormethan, 92,7%ee)

**Beispiel 5: (4S,5S)-4,5-Bis-(3'-benzyloxy-4'-methoxyphenyl)-2-oxo-1,3,2-dioxathiolan**

**Ansatz:** 30,74g (63,2mmol) (1S,2S)-1,2-Bis-(3'-benzyloxy-4'-methoxyphenyl)-ethandiol ($[\alpha]_D$ = +120,3° (c = 1, $CH_2Cl_2$) 92,7%ee)
150ml Dichlormethan, abs.
33,5ml (240mmol) Triethylamin
6,9ml (94,6mmol) Thionylchlorid
in 10ml Dichlormethan abs.

**Durchführung:** Diol, gelöst in Dichlormethan und Triethylamin, wurde unter $N_2$ bei 0°C vorgelegt, Thionylchlorid, verdünnt mit Dichlormethan, wurde langsam zugetropft (exotherme Reaktion). Nach 40min bei 0°C erfolgte die Aufarbeitung (ebenfalls bei 0°C) durch Zugabe von 150ml Diethylether und anschließendem Ausschütteln, zweimal mit 75ml Wasser und einmal mit 75ml gesättigter NaCl-Lösung. Die organische Phase wurde über Magnesiumsulfat getrocknet, einrotiert, nochmals mit Dichlormethan durchrotiert und im Hochvakuum 1h getrocknet.

**Ausbeute:** quantitativ

**Anal.Daten:** $\mathbf{R_f}$(Dichlormethan/Methanol 98:2) = 0,76; $\mathbf{R_f}$(Edukt) = 0,24
$[\alpha]_D$ = 171,4° (c = 1, Dichlormethan)

**Beispiel 6: (4S,5S)-4,5-Bis-(3'-benzyloxy-4'-methoxyphenyl)-2,2-dioxo-1,3,2-dioxathiolan**

**Ansatz:** 33g (63mmol) (4S,5S)-4,5-Bis-(3'-benzyloxy-4'-methoxyphenyl)-2-oxo-1,3,2-dioxathiolan
300ml Dichlormethan/Acetonitril 1:1, 200ml Eiswasser
72,7mg (0,35mmol) Ruthenium(III)-chlorid Hydrat
26,83g (125,4mmol) Natriummetaperjodat

**Durchführung:** Zur Lösung des cycl. Sulfits in Dichlormethan/Acetonitril 1:1 wurde bei 0°C und unter Schutzgasatmosphäre schnell nacheinander Eiswasser, Ruthenium(III)chlorid und $NaJO_4$ zugegeben und bei dieser Temperatur 1h nachgerührt. Die Aufarbeitung erfolgte durch Zugabe von 150ml Diethylether, zweimaligem Ausschütteln mit je 75ml Wasser und einmaligem Ausschütteln mit 75ml gesättigter Natriumchlorid-Lösung. Die organische Phase wurde über Magnesiumsulfat getrocknet.

**Anal.Daten:** $[\alpha]_D$ = 183,6 (c = 1, Dichlormethan)

**Verfahrensstufe (a):**

**Beispiel 7: rel-(1S,2R)-1,2-Bis-(3'-benzyloxy-4'-methoxyphenyl)-2-[(2'',2''-dimethoxyethyl)-methylamino]-ethanol**

**Ansatz:** 51,7g (0,11 mol) rel(S,S)-3,3'-Dibenzyloxy-4,4'-dimethoxystilbenoxid
1800ml Dichlormethan
100ml 2-Butanol
25ml (0,19mol) Methylaminoacetaldehyddimethylacetal
5g Kieselgel

**Durchführung:** Das Stilbenoxid, gelöst in Dichlormethan und 2-Butanol, wurde zusammen mit Methylaminoacetaldeyddimethylacetal und Kieselgel langsam (ca.8h) auf 120°C erhitzt, wobei zuerst Dichlormethan, dann Butanol abdestillierte. Die Aufarbeitung erfolgte durch Filtration und zweimaliges Ausschütteln mit Wasser/Dichlormethan. Die organische Phase wurde zweimal mit Toluol einrotiert und chromatographisch (Dichlormethan/Methanol 98:2) gereinigt.

**Ausbeute:** 50,9g (79%)

**Anal.Daten:** $\mathbf{R_f}$(Dichlormethan/Methanol 97:3) = 0,36
$\mathbf{R_f}$(Edukt, Dichlormethan) = 0,37

**Beispiel 8: rel-(1S,2R)-1,2-Bis-(3'-benzyloxy-4'-methoxyphenyl)-2-[(2'',2''-dimethoxyethyl)-methylamino]-ethanol**

**Ansatz:** 4,68g (10mmol) rel-(S,S)-3,3'-Dibenzyloxy-4,4'-dimethoxystilbenoxid
3,57g (3,83ml, 30mmol) Methylaminoacetaldehyddimethylacetal
3,41g (3,57ml, 12mmol) Titan(IV)-tetraisopropylat
200ml Dichlormethan, wasserfrei

**Durchführung:** Zu Epoxid und Methylaminoacetaldehyddimethylacetal, gelöst in wasserfreiem Dichlormethan, wurde Titan(IV)-tetraisopropylat, gelöst in wenig Dichlormethan, bei -20°C langsam zugetropft. Nach 30min wurde auf Raumtemperatur erwärmt und noch 2h nachgerührt. Die Aufarbeitung erfolgte durch Waschen der organischen Phase mit gesätt. Natriumhydrogencarbonat-Lösung. Nach fünfmaligem Waschen mit Wasser und abschließend mit gesätt. Natriumchlorid-Lösung wurde einrotiert. Das gewünschte Produkt wurde in Form eines Öls erhalten.

**Ausbeute:** 4,79g (83%)

**Anal.Daten:** $\mathbf{R_f}$(Dichlormethan/Methanol 97:3) = 0,36; $\mathbf{R_f}$(Edukt) = 0,85

**Beispiel 9: (1S,2R)-1,2-Bis-(3'-benzyloxy-4'-methoxyphenyl)-2-[(2'',2''-dimethoxyethyl)-methylamino]-ethanol**

**Ansatz:** ca. 33g (63mmol) (4S,5S)-4,5-Bis-(3'-benzyloxy-4'-methoxyphenyl)-2,2-dioxo-1,3,2-dioxathiolan
300 ml Dichlormethan
50ml 2-Butanol
48,4ml (377mmol) Methylaminoacetaldehyddimethylacetal

**Durchführung:** Das cycl. Sulfat, gelöst in Dichlormethan und 2-Butanol, wurde zusammen mit Methylaminoacetaldeyddimethylacetal langsam (ca.8h) auf 150°C erhitzt, wobei zuerst Dichlormethan, dann Butanol abdestillierte. Das Reaktionsgemisch wurde 1h bei dieser Temperatur gehalten, mit 20%iger wässr.Schwefelsäure versetzt und noch 30min bei 0°C gerührt. Danach wurde mit 20% Natriumhydroxid-Lösung auf pH = 10 gebracht und mit Dichlormethan extrahiert. Die weitere Reinigung kann chromatographisch erfolgen (Dichlormethan/Methanol 97:3)

**Ausbeute:** 18,2g (57%)
**Anal.Daten:** $R_f$(Dichlormethan/Methanol 97:3) = 0,39
$[\alpha]_D$ = -111,2° (c = 1, Dichlormethan)

**Beispiel 10: (1S,2R)-1,2-Bis-(3'-benzyloxy-4'-methoxyphenyl)-2-[(2'',2''-dimethoxyethyl)-amino]-ethanol**

**Ansatz:** 187,2g (0,4mol) rel-(S,S)-3,3'-Dibenzyloxy-4,4'-dimethoxystilbenoxid
210g (2mol) Aminoacetaldehyddimethylacetal
5l Dichlormethan
3l Ethanol

**Durchführung:** Das Stilbenoxid, gelöst in Dichlormethan und Ethanol, wurde zusammen mit Methylaminoacetaldeyddimethylacetal langsam (ca.8h) auf 150°C erhitzt, wobei zuerst Dichlormethan und dann Ethanol bis zu einem Restvolumen von 1,3 l abdestilliert wurden. Die Aufarbeitung erfolgte durch dreimaliges Ausschütteln mit Wasser/Dichlormethan. Nach Entfernen des Lösungsmittels kristallisierte das Produkt aus und wurde abgesaugt.

**Ausbeute:** 132g (57,6%)
**Anal.Daten:** $R_f$(Dichlormethan/Methanol 97:3) = 0,26; $R_f$(Edukt) = 0,86
**Smp.** 117°C

**Beispiel 11: a) rel-(1S,2R)-1,2-Bis-(3'-benzyloxy-4'-methoxyphenyl)-2-aminoethanol**

**Ansatz:** 1,0g (2,1mmol) rel(S,S)-3,3'-Dibenzyloxy-4,4'-dimethoxystilbenoxid
15ml EtOH, abs.
15ml Ammoniak

**Durchführung:** Ammoniak wurde in eine Vorlage des Epoxids in Ethanol einkondensiert Nach 7h bei 80°C im Autoklaven wurde das Reaktionsgemisch einrotiert und chromatographiert (Dichlormethan/Methanol 9:1).

**Ausbeute:** 0,68g (67%)
**Anal.Daten:** $R_f$(Dichlormethan/Methanol 9:1)- 0,36; $R_f$(Edukt) = 0,90

**Beispiel 11: b) rel-(4R,5S)-4,5-Bis-(3'-benzyloxy-4'-methoxyphenyl)-oxazolidin-2-on**

**Ansatz:** 1,0g (2,06mmol) rel-(1S,2R)-1,2-Bis-(3'-benzyloxy-4'-methoxyphenyl)-2-amino-ethanol
334mg (2,05mmol) Carbonyldiimidazol
20ml Toluol

**Durchführung:** Das Reaktionsgemisch wurde 2h unter Rückfluß erhitzt. Die Aufarbeitung erfolgte durch Einrotieren, die Reinigung durch Chromatographie (Dichlormethan/Methanol 95:5).

**Ausbeute:** 905mg (86%)
**Anal.Daten:** $R_f$(Dichlormethan/Methanol 9:1) = 0,53; $R_f$(Edukt) = 0,08

**Beispiel 11: c) rel-(4R,5S)-4,5-Bis-(3'-benzyloxy-4'-methoxyphenyl)-3-(2'-hydroxyethyl)-oxazolidin-2-on**

**Ansatz:** 100mg rel-(4R,5S)-4,5-Bis-(3'-benzyloxy-4'-methoxyphenyl)-oxazolidin-2-on
5ml Ethylenoxid
15ml Tetrahydrofuran
5mg Methylammoniumchlorid

**Durchführung:** Das Reaktionsgemisch wurde 10h hei einer Temperatur von 120 °C in einem Druckgefäß gerührt. Danach wurde einrotiert und chromatographiert. (Dichlormethan/Methanol 95:5).

**Ausbeute:** 80mg (73%)

**Anal.Daten:** $\mathbf{R_f}$(Dichlormethan/Methanol 95:5) = 0,62; $\mathbf{R_f}$(Edukt) = 0,75

**Beispiel 12: a) rel-(1S,2R)-1,2-Bis-(3'-benzyloxy-4'-methoxyphenyl)-2-methylamino-ethanol**

**Ansatz:** 39g (80mmol) rel-(S,S)-3,3'-Dibenzyloxy-4,4'-dimethoxystilbenoxid
250ml Methylamin (30% in Methanol)
100ml Dichlormethan

**Durchführung:** Das Reaktionsgemisch wurde bei einer Manteltemperatur von 100 °C in einem Schwenkautoklaven mit 500ml Fassungsvermögen 20h geschwenkt. Die Aufarbeitung erfolgte durch Einrotieren der Reaktionslösung und dreimaliges Ausschütteln mit Wasser/Dichlormethan. Anschließend wurde das Rohprodukt chromatographiert (Dichlormethan/Methanol 95:5).

**Ausbeute:** 32,0g(64%)

**Anal.Daten:** $\mathbf{R_f}$(Dichlormethan/Methanol 95:5) = 0,25; $\mathbf{R_f}$(Edukt) = 0,09

**Beispiel 12: b) rel-(1S,2R)-1,2-Bis(3'-benzyloxy-4'-methoxyphenyl)-2-[(2",2"-dimethoxyethyl)-methylamino]-ethanol**

**Ansatz:** 1,40g (2,8mmol) rel-(1S,2R)-1,2-Bis-(3'-benzyloxy-4'-methoxyphenyl)-2-methylamino-ethanol
0,43ml (0,61g, 3,6mmol) Bromacetaldehyddimethylacetal
0,71g (8,4mmol) Natriumhydrogencarbonat

**Durchführung:** Das Methylaminoethanol wurde zusammen mit dem Bromacetaldehyddimethylacetal und Natriumhydrogencarbonat 4h auf 130 °C erwärmt. Der Rückstand wurde in Dichlormethan aufgenommen, die Lösung wurde zuerst mit Natriumhydrogencarbonat- und dann mit Natriumchloridlösung gewaschen und einrotiert. Zur Reinigung kann chromatographiert werden.

**Ausbeute:** 1,43g (87%)

**Anal.Daten:** $\mathbf{R_f}$(Dichlormethan/Methanol 9:1) = 0,85; $\mathbf{R_f}$(Edukt) = 0,28

**Beispiel 13: rel-(1S,2R)-1,2-Bis-(3',4'-dimethoxyphenyl)-2-[(2",2"-dimethoxyethyl)-amino]-ethanol**

**Ansatz:** 57g (0,17mol) 3,3',4,4'-Tetramethoxystilbenoxid
120ml (1,1mol)Aminoacetaldehyddimethylacetal
1,51 Dichlormethan, 80ml 2-Butanol

**Durchführung:** Das Reaktionsgemisch wurde unter einer Schutzgasatmosphäre langsam bis auf eine Temperatur von 130 °C erhitzt, wobei die Lösungsmittel abdestillierten. Danach wurde eine Stunde bei dieser Temperatur gerührt. Die Aufarbeitung erfolgte durch mehrmaliges Ausschütteln mit Dichlormethan/Wasser. Die organische Phase wurde einrotiert und durch Säulenchromatographie (Dichlormethan/Methanol 98:2) gereinigt.

**Ausbeute:** 63,0g (88%)

**Anal.Daten:** $\mathbf{R_f}$(Dichlormethan/Methanol 95:5) = 0,32; $\mathbf{R_f}$(Edukt) = 0,90

**Beispiel 14: 2-(3',4'-Dimethoxyphenyl)-2-[(2",2"-dimethoxyethyl)-amino]-ethanol**
**1-(3',4'-Dimethoxyphenyl)-2-[(2",2"-dimethoxyethyl)-amino]-ethanol**

**Ansatz:** 28,6g (0,16mol) 3,4-Dimethoxystyroloxid
50ml Aminoacetaldehyddimethylacetal
5g Kieselgel
500ml Dichlormethan, 100ml Ethanol

**Durchführung:** Das Reaktionsgemisch wurde unter einer Schutzgasatmosphäre langsam bis auf eine Temperatur von 130 °C erhitzt, wobei die Lösungsmittel abdestillierten. Danach wurde eine Stunde bei dieser Temperatur gerührt. Die Aufarbeitung erfolgte durch mehrmaliges Ausschütteln mit Dichlormethan/Wasser. Die organische Phase wurde einrotiert. Die Diastereomeren (1:1) wurden chromatographisch (Dichlormethan/Methanol 95:5) getrennt.

**Gesamtausbeute:** 29,6g (65%)

**Anal.Daten:** $\mathbf{R_f}$(Dichlormethan/Methanol 95:5) = 0,10 und 0,18;
$\mathbf{R_f}$(Edukt) = 0,90

**Verfahrensstufe (b):**

**Beispiel 15: rel-(1'S,2'R)-Benzoesäure-[1',2'-bis-(3''-benzyloxy-4''-methoxy-phenyl)-2'-((2'''-dimethoxyethyl)-methylamino)-ethyl]ester**

**Ansatz:** 86,6g (0,15mol) (1S,2R)-1,2-Bis-(3'-benzyloxy-4'-methoxy-phenyl)-2-[(2'',2''-dimethoxyethyl)-methylamino-ethanol
600ml Dichlormethan, abs.
103ml (1,27mol) Pyridin
103ml (0,67mol) Benzoylchlorid

**Durchführung:** Unter Luftausschluß wurde das Reaktionsgemisch 18h bei RT gerührt. Die Aufarbeitung erfolgte durch Einrotieren am Hochvakuum bei 60°C und zweimaliges Durchspülen mit Toluol und Dichlormethan. Das Rohprodukt wurde in Dichlormethan aufgenommen und mit gesättigter wässriger Natriumhydrogencarbonat-Lösung vorsichtig ausgerührt. Die organische Phase wurde noch zweimal mit Natriumhydrogencarbonat-Lösung ausgeschüttelt, einrotiert und chromatographiert (Dichlormethan/Methanol 99:1).

**Ausbeute:** 59g (62%)
**Anal.Daten:** $\mathbf{R_f}$(Dichlormethan/Methanol 98:2) = 0,4; $\mathbf{R_f}$(Edukt) = 0,16

**Beispiel 16: a) rel-(1'S,2'R)-Essigsäure-[1',2'-bis-(3''-benzyloxy-4''-methoxy-phenyl)-2'-((2'''-dimethoxyethyl)-methylamino)-ethyl]ester**

**Ansatz:** 25,4g (44,3mmol) (1S,2R)-1,2-Bis-(3'-benzyloxy-4'-methoxy-phenyl)-2-[(2'',2''-dimethoxyethyl)-methylamino]-ethanol
500ml Dichlormethan, abs.
20ml (248mmol) Pyridin
24ml (254mmol) Essigsäureanhydrid

**Durchführung:** Unter Luftausschluß wurde das Reaktionsgemisch 18h bei RT gerührt. Die Aufarbeitung erfolgte durch Einrotieren des Rohproduktes im Hochvakuum bei 60°C, je zweimaliges Aufnehmen in Toluol und Dichlormethan und Einrotieren der organischen Phase. Zur Reinigung kann chromatographiert werden (Dichlormethan/Methanol 98:2).

**Ausbeute:** 21,8g (80%)
**Anal.Daten:** $\mathbf{R_f}$(Dichlormethan/Methanol 97:3) = 0,53; $\mathbf{R_f}$(Edukt) = 0,34

**Beispiel 16: b) (1'S,2'R)-Essigsäure-[bis-(3''-benzyloxy-4''-methoxy-phenyl)-2'-((2'''-dimethoxyethyl)-methylamino)-ethyl]ester**

Die Umsetzung wurde wie für die racemische Verbindung beschrieben durchgeführt.
$[\alpha]_D$ = -41,3° (c = 1, Dichlormethan, ee = 92,7%)

**Beispiel 17: rel-(4R,5S)-4,5-Bis-(3'-benzyloxy-4'-methoxyphenyl)-3-(2',2'-dimethoxyethyl)-oxazolidin-2-on**

**Ansatz:** 500mg (0,87mmol) rel-(1S,2R)-1,2-Bis-(3'-benzyloxy-4'-methoxyphenyl)-2-[(2',2'-dimethoxyethyl)-amino]-ethanol
320mg (2,0mmol) Carbonyldiimidazol
20ml Toluol

**Durchführung:** Das Reaktionsgemisch wurde eine Stunde unter Rückfluß erhitzt und einrotiert. Das Rohprodukt wurde in Dichlormethan aufgenommen, zweimal mit Wasser und einmal mit Natriumchlorid-Lösung ausgeschüttelt. Die organische Phase wurde einrotiert und chromatographiert (Dichlormethan/Methanol 97:3).

**Ausbeute:** 337mg (63%)
**Anal.Daten:** $\mathbf{R_f}$(Dichlormethan/Methanol 97:3) = 0,6; $\mathbf{R_f}$(Edukt) = 0,1

**Beispiel 18: rel-(1'S,2'R)-Essigsäure-[1',2'-bis-(3''-benzyloxy-4''-methoxyphenyl)-2'-(acetyl-(2''',2'''-dimethoxyethyl)-amino)-ethyl]ester**

**Ansatz:** 6,35g (10mmol) rel-(1S,2R)-1,2-Bis-(3'-benzyloxy-4'-methoxyphenyl)-2-[(2',2'-dimethoxyethyl)-amino]-ethanol
100ml Dichlormethan, abs.
8ml (99,2mmol) Pyridin
8,8ml (93,2mmol) Essigsäureanhydrid

**Durchführung:** Das Reaktionsgemisch wurde unter Luftausschluß 18h bei RT gerührt. Die Aufarbeitung erfolgte durch Einrotieren am Hochvakuum bei 60 °C, zweimaliges Einrotieren mit Toluol und säulenchromatographische Isolierung (Dichlormethan/Methanol 99:1) des Produktes.

**Ausbeute:** 6,91g (95%)
**Anal.Daten:** $\mathbf{R_f}$(Dichlormethan/Methanol 97:3) = 0,58; $\mathbf{R_f}$(Edukt) = 0,46

**Beispiel 19: rel-(1'S,2'R)-Pivalinsäure-[1',2'-bis-(3''-benzyloxy-4''-methoxyphenyl)-2'-((2''',2'''-dimethoxyethyl)-pivaloylamino)-ethyl]ester**

**Ansatz:** 5,0g (8,73mmol) rel-(1S,2R)-1,2-Bis-(3'-benzyloxy-4'-methoxyphenyl)-2-[(2',2'-dimethoxyethyl]-amino]-ethanol
15ml (187mmol) Pyridin
15ml (122mmol) Pivalinsäurechlorid

**Durchführung:** Das Säurechlorid wurde zum Aminoethanol, gelöst in Pyridin, zugetropft und 18h bei RT gerührt. Die Aufarbeitung erfolgte durch Einrotieren bei 0.01mmHg hei 60 °C, zweimaligem Einrotieren mit Toluol und Dichlormethan. Die abschließende Reinigung erfolgte chromatographisch (Dichlormethan/Methanol 98:2).

**Ausbeute:** 5,70g (88%)
**Anal.Daten:** $\mathbf{R_f}$(Dichlormethan/Methanol 97:3) = 0,32; $\mathbf{R_f}$(Edukt) = 0,08

**Beispiel 20: (2R,1'S,2'R)-O-Pivaloyl-mandelsäure-[1',2'-bis-(3''-benzyloxy-4''-methoxy-phenyl)-2'-((2''',2'''-dimethoxyethyl)-methylamino)-ethyl]ester**
**(2R,1'R,2'S)-O-Pivaloyl-mandelsäure-[1',2'-bis-(3''-benzyloxy-4''-methoxy-phenyl)-2'-((2''',2'''-dimethoxyethyl)-methylamino)-ethyl]ester**

**Ansatz:** 7,0g (11,9mmol) rel-(1S,2R)1,2-Bis-(3'-benzyloxy-4'-methoxy-phenyl)-2-((2',2'-dimethoxyethyl)-methylamino)-ethanol
3,0g (11.8mmol) R-Pivaloylmandelsäurechlorid
100ml Pyridin

**Durchführung:** Pivaloylmandelsäurechlorid wurde bei O °C zur Lösung des Aminoethanols in Pyridin zugetropft. Anschließend wurde 1h bei Raumtemperatur gerührt, einrotiert und chromatographiert (Dichlormethan/Methanol 99:1).

**Ausbeute:** 9,1g (94%)
**Anal.Daten:** $\mathbf{R_f}$(Dichlormethan/Methanol 97:3)
(2R,1'S,2'R)-Isomer: $\mathbf{R_f}$ = 0,75; $[\alpha]_D = -9°$ (c = 1, Dichlormethan)
(2R,1'R,2'S)-Isomer: $\mathbf{R_f}$ = 0,66; $[\alpha]_D = +2,7°$ (c = 1, $CH_2Cl_2$)
Edukt: $\mathbf{R_f}$ = 0,42

**Beispiel 21: Essigsäure.[2'-(3'',4''-dimethoxyphenyl)-2'-(acetyl-(2'''-dimethoxyethyl)-amino)ethyl]-ester**

**Ansatz:** 5,0g (17,5mmol) 2-(3',4'-Dimethoxyphenyl)-2-[(2'',2''-dimethoxyethyl)-amino]-ethanol
20ml Pyridin
20ml Essigsäureanhydrid

**Durchführung:** Das Reaktionsgemisch wurde unter Luftausschluß 3h bei Raumtemperatur gerührt. Die Aufarbeitung erfolgte durch zweimaliges Einrotieren mit Toluol im Hochvakuum bei 60 °C und säulenchromatographische Isolierung (Dichlormethan/Methanol 95:5) des Produktes.

**Ausbeute:** 5,45g (85%)
**Anal.Daten:** $\mathbf{R_f}$(Dichlormethan/Methanol 9:1) = 0,40; $\mathbf{R_f}$(Edukt) = 0,30

**Beispiel 22: rel-(5R,6S)-5,6-Bis-(3'-benzyloxy-4'-methoxyphenyl)-4-(2',2'-dimethoxyethyl)-1,4-oxazin-2,3-dion**

**Ansatz:** 200mg (0,35mmol) rel-(1S,2R)-1,2-Bis-(3'-benzyloxy-4'-methoxyphenyl)-2-((2',2'-dimethoxyethyl)-amino)-ethanol
50mg (0,39mmol) Oxalylchlorid
30mg (0,39mmol) Pyridin
20ml Dichlormethan, abs.

**Durchführung:** Zum Aminoethanol gelöst in Pyridin und Dichlormethan wurde Oxalylchlorid in Dichlormethan zugetropft. Nach 1h wurde einrotiert und mit Dichlormethan/gesätt. Natriumhydrogencarbonat-Lösung ausgeschüttelt. Die organische Phase wurde einrotiert und chromatographiert (Dichlormethan/Methanol 98:2)

**Ausbeute:** 200mg (91%)
**Anal.Daten:** $R_f$(Dichlormethan/Methanol 97:3) = 0,69; $R_f$(Edukt) = 0,36

**Beispiel 23: rel(1R,2R)-Pivalinsäure[1',2'-bis-(3'',4''-dimethoxyphenyl)-2-methylaminoethyl]ester**

**Ansatz:** 2,15g (5mmol) rel-(1R,2S)-Pivalinsäure-[1,2-Bis-(3',4'-dimethoxyphenyl)-2-ethanol]amid
2ml (2,97g, 14,1mmol) Trifluoressigsäureanhydrid
18ml Trifluoressigsäure

**Durchführung:** Das Pivaloylamid wurde im Gemisch Trifluoessigsäure/Trifluoressigsäureanhydrid 2h unter Rückfluß gekocht. Nach Abkühlen auf Raumtemperatur wurde Eis zugegeben, mit Kaliumcarbonat alkalisch gestellt und mit Dichlormethan extrahiert. Das Produkt wurde chromatographisch gereinigt.

**Ausbeute:** 1,78g (83%)
**Anal.Daten:** $R_f$(Dichlormethan/Methanol 97:3) = 0,40; $R_f$(Edukt) = 0,28

**Verfahrensstufe (c):**

**Beispiel 24: a) rel-(αS,1'R)-Essigsäure-[α-(7'-benzyloxy-4'-hydroxy-6'-methoxy-1',2',3',4'-tetrahydroisochinolin-1'-yl)-3-benzyloxy-4-methoxybenzyl]-ester**

**Ansatz:** 27,4g (44,3mmol) rel-(1'S,2'R)-Essigsäure-[bis-(3''-benzyloxy-4''-methoxyphenyl)-2'-((2'''-dimethoxyethyl)-methylamino)-ethyl]ester
102ml Aceton, 68 ml konz.Salzsäure

**Durchführung:** Zum Aminoethanol, gelöst in Aceton, wurde bei -20°C unter Schutzgasatmosphäre langsam die konz. Salzsäure zugetropft ($T^{max}$ 10°C). Danch wurde die Kühlung entfernt und noch ca. 8h bei RT gerührt; DC-Kontrolle. Die Reaktion wurde dünnschichtchromatographisch verfolgt und bei vollständiger Umsetzung der Edukts sofort abgebrochen. Die Aufarbeitung erfolgte durch Zugabe von Dichlormethan und Neutralisation mit konz. Ammoniaklösung. Anschließend wurde gesätt. Natriumhydrogencarbonat-Lösung zugegeben, die organische Phase wird abgetrennt und noch zweimal mit derselben Natriumhydrogencarbonat-Lösung ausgeschüttelt und einrotiert. Das Rohprodukt wurde über eine Filtriersäule gereinigt(Dichlormethan/Methanol 98:2).

**Ausbeute:** 22,1g (87%)
**Anal.Daten:** $R_f$(Dichlormethan/Methanol 97:3) 0,25 4'S-Isomer; 0,42 4'R-Isomer; 0,50 Edukt

**Beispiel 24: b) (αS,1'R,4'S)-Essigsäure-[α-(7'-benzyloxy-4'-hydroxy-6'-methoxy-1',2',3',4'-tetrahydroisochinolin-1'-yl)-3-benzyloxy-4-methoxybenzyl]-ester**

Die Verbindung wurde wie für das Racemat beschrieben hergestellt.
$[\alpha]_D$ = -2,3° (c = 1, Dichlormethan); ee = 92,7°

**Beispiel 25: rel-(αS,1'R)-Essigsäure-[α-(7'-benzyloxy-4'-hydroxy-6'-methoxy-1',2',3',4'-tetrahydroisochinolin-1'-yl)-3-benzyloxy-4-methoxybenzyl]-ester**
**rel-(αS,1'R)-Essigsäure-(α-(7'-benzyloxy-4',6'-dimethoxy-1',2',3',4'-tetrahydro-isochinolin-1'-yl)-3-benzyloxy-4-methoxybenzyl]-ester**

**Ansatz:** 3,14g (5mmol) rel-(1'S,2'R)-Essigsäure-(bis-(3''-benzyloxy-4''-methoxyphenyl)-2'-((2'''-dimethoxyethyl)-methyl-amino)-ethyl]ester
0,61ml (0,71g, 5mmol) Bortrifluorid-Diethylether-Komplex 100ml Dichlormethan, wasserfrei

**Durchführung:** Bortrifluoridetherat wurde bei -20°C zur Lösung des Acetals in wasserfreiem Dichlormethan langsam eingetropft. Nach Erwarmung auf Raumtemperatur wurde noch 1h gerührt, die Dichlormethanlösung wurde anschließend mit Natriumhydrogencarbonat-Lösung gewaschen und eingeengt. Nach Chromatographie wurde das Produktgemisch als zähes Öl erhalten.

**Ausbeute:** 1,98g (67%)

**Anal. Daten:** **$R_f$**(Dichlormethan/Methanol 97:3)α-Hydroxy-Prod.: 0,42; β-Hydroxy-Prod.: 0,25; α- und β-Methoxy-Prod.: ca. 0,45; Edukt: 0,50

**Beispiel 26: rel-(4R,5S)-5-(3''-Benzyloxy-4''-methoxyphenyl)-(7'-benzyloxy-6'-dimethoxy-1',2',3',4'-tetrahydro-isochinolino)-[1,2-c]-oxazolidin-2-on rel-(4R,5S)-5-(3''-Benzyloxy-4''-methoxyphenyl)-(7'-benzyloxy-4'-hydroxy-6'-methoxy-1',2',3',4'-tetrahydro-isochinolino)-[1,2-c]-oxazolidin-2-on**

**Ansatz:** 3,5g (5,7mmol) rel-(4R,5S)-4,5-Bis-(3'-benzyloxy-4'-methoxyphenyl)-3-(2',2'-dimethoxyethyl)-oxazolidin-2-on 100ml Aceton, 66,7ml konz. Salzsäure

**Durchführung:** Zum Acetal, gelöst in Aceton, wurde bei -20 ° C unter Schutzgasatmosphäre vorsichtig konz. Salzsäure zugetropft ($T^{max.}$ = 40 ° C). Das Reaktionsgemisch wurde 5h bei Raumtemperatur gerührt. Die Aufarbeitung erfolgte durch Zugabe von Dichlormethan, Neutralisation mit konz. Ammoniak-Lösung unter Eiskühlung und anschließende Zugabe von gesätt. Natriumhydrogencarbonat-Lösung. Die organische Phase wurde noch zweimal mit Natriumhydrogencarbonat-Lösung ausgeschüttelt und einrotiert. Das Produktgemisch kann chromatographisch gereinigt werden (Dichlormethan/Methanol 97:3)

**Ausbeute:** 2,26g (68%)

**Anal.Daten:** **$R_f$**(Produktgemisch; Dichlormethan/Methanol 97:3) = 0,51-0,2
**$R_f$**(Edukt) = 0,70

**Beispiel 27: rel-(αS,1'R)-Essigsäure-[α-(2'-acetyl-7'-benzyloxy-4'-hydroxy-6'-methoxy-1',2',3',4'-tetrahydro-isochinolin-1'-yl)-3-benzyloxy-4-methoxybenzyl]-ester**

**Ansatz:** 3,68g (5,6mmol) rel-(1'S,2'R)-Essigsäure-(bis-(3''-benzyloxy-4''-methoxyphenyl)-2'-(acetyl-(2''',2'''-dimethoxyethyl)-amino)-ethyl]ester
100ml Tetrahydofuran, 66,7ml konz. Salzsäure

**Durchführung:** Zum Aminoacetal, gelöst in Tetrahydrofuran, wurde bei -10 ° C unter Schutzgasatmosphäre langsam konz. Salzsäure zugetropft. Die Kühlung wurde entfernt und anschließend noch etwa 2.5h bei Raumtemperatur gerührt. Die Reaktion wurde dünnschichtchromatographisch verfolgt. Sofort nach vollständiger Umsetzung des Acetals wurde Dichlormethan zugegeben, unter Eiskühlung mit konz. Ammoniak neutralisiert, dann Natriumhydrogencarbonat-Lösung zugegeben und noch zweimal mit gesättigter wässriger Natriumhydrogencarbonat-Lösung gewaschen. Die organische Phase wurde einrotiert, das Produkt (Atropisomere!) wurde chromatographisch gereinigt (Dichlormethan/Methanol 99:1).

**Ausbeute:** 2,15g (66%)

**Anal.Daten:** **$R_f$**(Dichlormethan/Methanol) = 0,56; **$R_f$**(Edukt) = 0,46;

**Beispiel 28: rel-(αS,1'R)-Benzoesäure-[α-(7'-benzyloxy-4'-hydroxy-6'-methoxy-1',2',3',4'-tetrahydro-isochinolin-1'-yl)-3-benzyloxy -4-methoxybenzyl]-ester**

**Ansatz:** 10,4g (15mmol) rel-(1'S,2'R)-Benzoesäure-[bis-(3''-benzyloxy-4''-methoxyphenyl)-2'-((2'''-dimethoxyethyl)-methylamino)-ethyl]ester
446ml Aceton, 297ml konz. Salzsäure

**Durchführung:** Zum Acetal, gelöst in Aceton, wurde bei -20 ° C unter Schutzgasatmosphäre vorsichtig konz. Salzsäure zugetropft ($T^{max.}$ = 40 ° C). Das Reaktionsgemisch wurde 5h bei Raumtemperatur gerührt. Die Aufarbeitung erfolgte durch Zugabe von Dichlormethan, Neutralisation mit konz. Ammoniak-Lösung unter Eiskühlung und anschließende Zugabe von gesätt. Natriumhydrogencarbonat-Lösung. Die organische Phase wurde noch zweimal mit Natriumhydrogencarbonat-Lösung ausgeschüttelt und einrotiert. Das Produktgemisch kann chromatographisch gereinigt werden (Dichlormethan/Methanol 99:1)

**Ausbeute:** 5,4g (55,6%)

**Anal.Daten:** **$R_f$**(Dichlormethan/Methanol 98:2) = 0,43 (β-Isomer); 0,28 (α-Isomer); 0,72 (Edukt)

**Beispiel 29: rel-(αS,1'R,4'S)-3-Benzyloxy-4-methoxy-α-(7'-benzyloxy-4'-hydroxy-6'-methoxy-1',2',3',4'-tetrahydro-isochinolin-1'-yl)-benzylalkohol**

**Ansatz:** 10,0g (15,9mmol) rel-(1'S,2'R)-Essigsäure-[1',2'-bis-(3''-benzyloxy-4''-methoxy-phenyl)-2'-(2'''-dimethoxyethylmethyl-amino)-ethyl]ester

400ml Aceton
267ml konz. Salzsäure

**Durchführung:** Zum Acetal, gelöst in Aceton, wurde bei -20 °C unter Schutzgasatmosphäre vorsichtig konz. Salzsäure zugetropft ($T^{max.}$ = 40 °C). Das Reaktionsgemisch wurde 6h bei Raumtemperatur gerührt. Die Aufarbeitung erfolgte durch Zugabe von etwas Aceton und 50%iger NaOH bis pH = 12. Dabei stieg die Reaktionstemperatur auf 60 °C an. Bei dieser Temperatur wurde eine Stunde nachgerührt, dann wurde das Volumen auf ein Drittel eingeengt. Nach Zugabe von Dichlormethan wurde mit konz. Salzsäure neutralisiert, anschließend Natriumhydrogencarbonat-Lösung zugegeben, dreimal mit Dichlormethan extrahiert, einrotiert und chromatographiert.

**Ausbeute:** 5,26g (61%)
**Anal.Daten:** $\mathbf{R_f}$(Dichlormethan/Methanol 95:5) = 0,21; $\mathbf{R_f}$(Edukt) = 0,90

**Beispiel 30: Essigsäure-(2'-acetyl-6',7'-dimethoxy-1',2'-dihydro-isochinolin-1'-yl)-methyl]-ester**

**Ansatz:** 500mg (1,35mmol) Essigsäure-(2'-(3'',4''-dimethoxyphenyl)-2'-(acetyl-(2'''-dimethoxyethyl)-amino)ethyl]ester 10ml Aceton, 5ml konz. Salzsäure

**Durchführung:** Zum acylierten Aminoacetal, gelöst in Aceton, wurde bei 0 °C unter Schutzgasatmosphäre langsam konz. Salzsäure zugetropft. Die Kühlung wurde entfernt, anschließend wurde noch 2h bei Raumtemperatur gerührt. Die Reaktion wurde dabei dünnschichtchromatographisch verfolgt. Sofort nach vollständiger Umsetzung des Acetals wurde Dichlormethan zugegeben, unter Eiskühlung mit konz. Ammoniak neutralisiert und dann Natriumhydrogencarbonat-Lösung zugegeben. Nach Phasentrennung wurde noch zweimal mit gesättigter, wässriger Natriumhydrogencarbonat-Lösung gewaschen. Die organische Phase wurde einrotiert, das Produkt wurde chromatographisch gereinigt (Dichlormethan/Methanol 95:5).

**Ausbeute:** 220mg (54%)
**Anal.Daten:** $\mathbf{R_f}$(Dichlormethan/Methanol 9:1) = 0,28; $\mathbf{R_f}$(Edukt) = 0,40 Atropisomere!

**Reduktionen mit komplexen Hydriden**

**Beispiel 31: 7-Benzyloxy-6-methoxy-1-(3'-benzyloxy-4'-methoxybenzyl)-2-methyl-1,2,3,4-tetrahydro-isochinolin**

**Ansatz:** 3,0g (5,54mmol) rel-(αS,1'R,4'S)-3-Benzyloxy-4-methoxy-α-(-7'-benzyloxy-4'-hydroxy-6'-methoxy-1'-1',2',3',4'-tetrahydro-isochinolin-1'-yl)-benzylalkohol
150ml Tetrahydrofuran, abs.
1,13ml (15,5mmol) Thionylchlorid
1,31ml (15mmol) Pyridin
1,57g (41,4mmol) Lithiumaluminiumhydrid

**Durchführung:** Zur Dihydroxyverbindung, gelöst in Tetrahydrofuran/Pyridin, wurde bei 0 °C unter Schutzgasatmosphäre Thionylchlorid zugetropft. Nach 30min bei 0 °C wurde die Kühlung entfernt und langsam auf Raumtemperatur erwärmt.Die dabei entstehende Suspension wurde 2h gerührt und wieder auf -20 °C abgekühlt. Zum Reaktionsgemisch wurde spatelweise Lithiumaluminiumhydrid zugegeben, wobei die Temperatur bis auf maximal 15 °C stieg. Die Kühlung wurde entfernt und noch 2h gerührt. Für die Aufarbeitung wurden vorsichtig 1.2ml Wasser, 1.2ml 15%ige NaOH und nochmals 3.6ml Wasser zugegeben. Der entstandene Rückstand wurde abfiltriert und ausgiebig mit heißem THF nachgewaschen. Das Filtrat wurde einrotiert und chromatographiert (Dichlormethan/Methanol 98:2).

**Ausbeute:** 1,80g (64%)
**Anal.Daten:** $\mathbf{R_f}$(Dichlormethan/Methanol 95:5 = 0,42; $\mathbf{R_f}$ = 0,40

**Beispiel 32: rel-(αS,1'R)-Essigsäure-[α-(7'-benzyloxy-6'-methoxy-1',2',3',4'-tetrahydro-isochinolin-1'-yl)-3-benzyloxy-4-methoxybenzyl]ester**

**Ansatz:** 500mg (0,86mmol) rel-(αS,1'R)-Essigsäure-[α-(1',2',3',4'-tetrahydro-7'-benzyloxy-4'-hydroxy-6'-methoxy-1'-isochinolinyl)-3-benzyloxy-4-methoxybenzyl]ester
80mg (1,03mmol) Pyridin
110mg (0,95mmol) Thionylchlorid
15ml Tetrahydrofuran

**Durchführung:** Zur Lösung der Ausgangsverbindung in Tetrahydrofuran wurden bei -20 °C Pyridin und Thionylchlorid zugetropft. Nach Erwärmung auf Raumtemperatur wurde noch 30min gerührt und auf 0 °C

abgekühlt. Anschließend tropfte man Natriumborhydrid, gelöst in Tetrahydrofuran, zu und rührte noch 1h. Zur Aufarbeitung wurde das Lösungsmittel zu 4/5 abrotiert, anschließend wurde mit Dichlormethan/gesätt. Natriumhydrogencarbonat-Lösung ausgeschüttelt. Die organische Phase wurde einrotiert und chromatographiert (Dichlormethan/Methanol 97:3)

**Ausbeute:** 0,30g (62%)
**Anal.Daten:** $\mathbf{R_f}$(Dichlormethan/Methanol 95:5) = 0,49; $\mathbf{R_f}$(Edukt) = 0,36

**katalytische Hydrierungen**

**Beispiel 33: 2-Acetyl-7-hydroxy-6-methoxy-(3'-hydroxy-4'-methoxybenzyl)-1,2,3,4-tetrahydro-isochinolin (N-Acetyl-Norreticulin)**

**Ansatz:** 250mg (0,42mmol) rel-(αS,1'R)-Essigsäure-[α-(2'-acetyl-7'-benzyloxy-6'-methoxy-1',2'-dihydro-isochinolin-1'-yl)-3-benzyloxy-4-methoxy-benzyl]ester
21ml Ethanol, 14ml dest. Wasser
250mg 10% Palladium auf Aktivkohle

**Durchführung:** Die Ausgangsverbindung wurde in einem VA-Rührautoklaven 18h bei 100°C und 85bar Wasserstoffdruck unter kräftigem Rühren hydriert. Die Aufarbeitung erfolgte durch Abfiltrieren des Katalysators, Einrotieren des Filtrats und Ausschütteln des Produktgemisches mit Dichlormethan/gesättigter Natriumhydrogencarbonat-Lösung. Die organische Phase wurde einrotiert und chromatographiert (Dichlormethan/Methanol 97:3)

**Ausbeute:** 90mg (51,4%)
**Anal.Daten:** $\mathbf{R_f}$(Dichlormethan/Methanol 95:5) = 0,35; $\mathbf{R_f}$(Edukt) = 0,48

**Beispiel 34: rel-(αS,1'R)-Benzoesäure-[α-(2'-benzoyl-7'-hydroxy-6'-methoxy-1',2',3',4'-tetrahydro-isochinolin-1'-yl)-3-hydroxy-4-methoxybenzyl]ester**

**Ansatz:** 1,43g (2,0mmol) rel-(αS,1'R)-Benzoesäure-[α-(7'-benzyloxy-6'-methoxy-1',2'-dihydro-isochinolin- 1'-yl)-3-benzyloxy-4-methoxy-benzyl]ester
180ml Isopropanol, 120ml dest. Wasser
1,16g 10% Palladium auf Aktivkohle
1,51g (16,7mmol) Oxalsäure

**Durchführung:** Die Ausgangsverbindung wurde im Schwenkautoklaven 18h bei 65°C und 150bar Wasserstoffdruck unter kräftigem Schwenken hydriert. Die Aufarbeitung erfolgte durch Abfiltrieren des Katalysators, Einrotieren des Filtrats und Ausschütteln des Produktgemisches mit Dichlormethan/gesättigter Natriumhydrogencarbonat-Lösung. Die organische Phase wurde einrotiert und chromatographiert (Dichlormethan/Methanol 97:3)

**Ausbeute:** 0,81g (75,3%)
**Anal.Daten:** $\mathbf{R_f}$(Dichlormethan/Methanol 95:5) = 0,55; $\mathbf{R_f}$ = 0,95

**Beispiel 35: 2-Benzoyl-7-hydroxy-6-methoxy-(3'-hydroxy-4'-methoxybenzyl)-1,2,3,4-tetrahydro-isochinolin (N-Benzoyl-norreticulin)**

**Ansatz:** 500mg (0,93mmol) rel-(αS,1'R)-Benzoesäure-[α-(2'-benzoyl-7'-hydroxyoxy-6'-methoxy-1',2',3',4'-tetrahydroisochinolin-1'-yl)-3-hydroxy-4-methoxybenzyl]ester
21ml Isopropanol
2ml dest. Wasser
12ml konz. Salzsäure
500mg 10% Palladium auf Aktivkohle

**Durchführung:** Die Ausgangsverbindung wurde im Autoklaven 18h bei 70°C und 85bar Wasserstoffdruck unter kräftigem Schwenken hydriert. Die Aufarbeitung erfolgte durch Abfiltrieren des Katalysators, Einrotieren des Filtrats und Ausschütteln des Produktgemischs mit Dichlormethan/gesättigter Natriumhydrogencarbonat-Lösung. Die organische Phase wurde einrotiert und chromatographiert (Dichlormethan/Methanol 95:5).

**Ausbeute:** 115mg (48,9%)
**Anal.Daten:** $\mathbf{R_f}$(Dichlormethan/Methanol 95:5) = 0,41; $\mathbf{R_f}$(Edukt) = 0,44

**Beispiel 36: a) 7-Hydroxy-(3'-hydroxy-4'-methoxybenzyl)-6-methoxy-2-methyl-1,2,3,4-tetrahydro-iso-**

**chinolin (Reticulin)**

**Ansatz:** 2,32g (3,71mmol) rel-(αS,1'R)-Essigsäure-[α-(4'-acetoxy-7'-benzyloxy-6'-methoxy-1',2',3',4'-tetrahydro-isochinolin-1'-yl)-3-benzyloxy-4-methoxybenzyl]ester
140ml Ethanol
93ml dest. Wasser
0,66g 10% Palladium auf Aktivkohle

**Durchführung:** Die Ausgangsverbindung wurde im 500ml Schwenkautoklaven 18h bei 70°C und 85bar Wasserstoffdruck unter kräftigem Schwenken hydriert. Die Aufarbeitung erfolgte durch Abfiltrieren des Katalysators, Einrotieren des Filtrats und mehrmaliges Ausschütteln des Produktgemischs mit Dichlormethan/gesättigter Natriumhydrogencarbonat-Lösung. Die organische Phase wurde einrotiert und chromatographiert (Dichlormethan/Methanol 9:1).

**Ausbeute:** 1,58g (88%)
**Anal.Daten:** $\mathbf{R_f}$(Dichlormethan/Methanol 9:1) = 0,07; $\mathbf{R_f}$(Edukt) = 0,90
**Reinheit:** 85,15% HPLC

**Beispiel 36: b) 1R-7-Hydroxy-(3'-hydroxy-4'-methoxybenzyl)-6-methoxy-2-methyl-1,2,3,4-tetrahydro-isochinolin (Reticulin)**

Die Verbindung wurde wie für das Racemat beschrieben hergestellt. Das Hydrochlorid wurde durch Einrotieren mit ethanolischer Salzsäure erhalten.

R-Reticulin-HCl: $[\alpha]_D$ = -85,3° (c = 0,82, Methanol; ee = 92%)
Literaturwert: $[\alpha]_D$ = -92,7° (c = 0,82, Methanol)

**Beispiel 37: 7-Hydroxy-(3'-hydroxy-4'-methoxybenzyl)-6-methoxy-2-methyl-1,2,3,4-tetrahydro-isochinolin (Reticulin)**

**Ansatz:** 300mg (0,5mmol) rel-(αS,1'R)-Essigsäure-[α-(4'-hydroxy-7'-benzyloxy-6'-methoxy-1',2',3',4'-tetrahydro-isochinolin-1'-yl)-3-benzyloxy-4-methoxybenzyl]ester
20ml Ethanol
13,7ml dest. Wasser
300mg 10% Palladium auf Aktivkohle
392mg (4,35mmol) Oxalsäure

**Durchführung:** Die Ausgangsverbindung wurde im Autoklaven 18h bei 70°C und 50bar Wasserstoffdruck unter kräftigem Schwenken hydriert. Die Aufarbeitung erfolgte durch Abfiltrieren des Katalysators, Einrotieren des Filtrats und mehrmaliges Ausschütteln des Produktgemischs mit Dichlormethan/gesättigter Natriumhydrogencarbonat-Lösung. Die organische Phase wurde einrotiert und chromatographiert (Dichlormethan/Methanol 9:1).

**Ausbeute:** 150mg (88,6%)
**Anal.Daten:** $\mathbf{R_f}$(Dichlormethan/Methanol 9:1) = 0,08; $\mathbf{R_f}$(Edukt)- 0,85
**Reinheit:** 79% HPLC

**Beispiel 38: 6,7-Dimethoxy-(3',4'-dimethoxybenzyl)-isochinolin (Papaverin)**

**Ansatz:** 200mg (0,45mmol) rel-(4R,5S)-5-(3'',4''-Dimethoxyphenyl)-(-4'-acetoxy-6',7'-dimethoxy-1',2',3',4'-tetrahydro-isochinolino)-[1,2-c]-oxazolidin-2-on
5ml abs. Toluol
10mg p-Toluolsulfonsäure

**Durchführung:** Das Reaktionsgemisch wurde 3h unter Rückfluß gekocht, die Reaktionslösung wurde mit gesätt. Natriumhydrogencarbonat-Lösung gewaschen, eingeengt und chromatographisch gereinigt.

**Ausbeute:** 120mg (79%)
**Anal.Daten:** $\mathbf{R_f}$(Dichlormethan/Methanol 95:5) = 0,53; $\mathbf{R_f}$(Edukt) = 0,65

**Beispiel 39: 6,7-Dimethoxy-1-methyl-isochinolin**

**Ansatz:** 100mg (0,33mmol) Essigsäure-(2'-acetyl6',7'-dimethoxy-1',2'-dihydroisochinolin-1'-yl)-methylester
4ml Ethanol

2ml konz. Salzsäure

**Durchführung:** Das Reaktionsgemisch wurde 2h unter Rückfluß gekocht, abgekühlt und mit Dichlormethan und gesätt. Natriumhydrogencarbonat-Lösung ausgeschüttelt. Die Reinigung erfolgte durch Chromatographie (Dichlormethan/Methanol 95:5).

**Ausbeute:** 37mg (55%)

**Anal.Daten:** $\mathbf{R_f}$(Dichlormethan/Methanol 9:1) = 0,55; $\mathbf{R_f}$(Edukt) = 0,43

**kombinierte Acylierung-Cyclisierungen:**

**Beispiel 40: rel-(αS,1'R)-Benzoesäure-[α-(2'-benzoyl-7'-benzyloxy-6'-methoxy-1',2'-dihydro-isochinol in-1'-yl)-3-benzyloxy-4-methoxybenzyl]ester**

**Ansatz:** 5,0g (8,7mmol) rel-(1S,2R)-1,2-Bis-(3'-benzyloxy-4'-methoxyphenyl)-2-((2',2'-dimethoxyethyl)-amino)-ethanol
6,08ml Benzoylchlorid (0,05mol)
4,25ml Pyridin (0,05mol)
20ml Dichlormethan, abs.

**Durchführung:** Das Reaktionsgemisch wurde unter Luftausschluß 18h bei RT gerührt. Die Aufarbeitung erfolgte durch Einrotieren im Hochvakuum bei 60°C, zweimaligem Einrotieren mit Toluol und Dichlormethan und Ausschütteln mit Dichlormethan/Wasser. Die organische Phase wurde einrotiert und über Nacht an der Luft stehen gelassen. Die Reinigung erfolgte durch Chromatographie (Dichlormethan/Methanol 99:1).

**Ausbeute:** 2,94g (59%)

**Anal.Daten:** $\mathbf{R_f}$(Dichlormethan/Methanol 99:1) = 0,29; $\mathbf{R_f}$(Edukt) = 0,24 Atropisomere!

**Aromatisierungen**

**Beispiel 41: 6,7-Dimethoxy-(3',4'-dimethoxybenzyl)-isochinolin (Papaverin)**

**Ansatz:** 400mg (0,35mmol) rel-(αS,1'R,4'S)-Benzoesäure-[α-(2'-benzoyl-6',7'-dimethoxy-1',2'-dihydro-isochinolin-1'-yl)-3,4-dimethoxybenzyl]ester
2,2g 85%ige Kaliumhydroxid-Lösung
0,90g (18mmol) Hydrazin-Hydrat, 50ml Ethylenglycol

**Durchführung:** Das Reaktionsgemisch wurde auf 160°C erhitzt und bei dieser Temperatur 90min unter einer Schutzgasatmosphäre gerührt. Zur Aufarbeitung wurde mit Wasser/Dichlormethan ausgeschüttelt. Die Reinigung erfolgte chromatographisch.

**Ausbeute:** 97mg (82%)

**Anal.Daten:** $\mathbf{R_f}$(Dichlormethan/Methanol 97:3) = 0,34; $\mathbf{R_f}$(Edukt) = 0,65

**Beispiel 42: 6,7-Dimethoxy-(3',4'-dimethoxybenzyl)-isochinolin (Papaverin)**

**Ansatz:** 2,30g (6mmol) rel-(4R,5S)-5-(3'',4''-Dimethoxyphenyl)-(6',7'-dimethoxy-1',2'-dihydro-isochinolino)-[1,2-c]-oxazolidin-2-on 50ml 6N Salzsäure, 10ml Methanol

**Durchführung:** Das Carbamat wurde im Gemisch Methanol/wässrige Salzsäure 5h unter Rückfluß gekocht. Nach Neutralisation mit konz. Ammoniak wurde mit Dichlormethan extrahiert und einrotiert.

**Ausbeute:** 1,79g (88%)

**Anal.Daten:** $\mathbf{R_f}$(Dichlormethan/Methanol 95:5) = 0,45; $\mathbf{R_f}$(Edukt) = 0,67

**Patentansprüche**

**1.** Verfahren zur Herstellung von 1-Alkyl-1,2,3,4-tetra-hydroisochinolinderivatender allgemeinen Formel

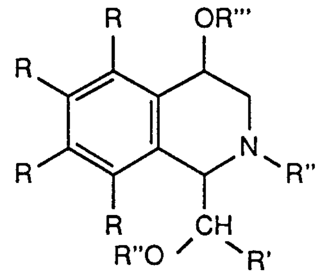

(1)

worin

R = H- oder Halogenatome, Alkylamino-, Dialkylamino-, Acylamino-, Hydroxy-, Alkoxy-, Aralkoxy- oder Acyloxyreste oder jeweils 2 der Reste R zusammen einen Alkylendioxyrest bilden;

R' = H-Atom oder Alkyl-, Acyl-, Aryl- oder mit Resten R substituierter Arylrest

R'' = H-Atome, Acyl-, Alkyl-, Aryl- oder Silylreste oder 2 der Reste R'' zusammen einen Diacylrest bilden;

R''' = H-Atome oder Alkylreste

dadurch gekennzeichnet, daß

(a) Verbindungen der allgemeinen Formeln

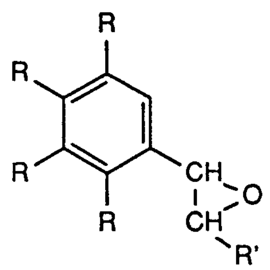

(2) oder

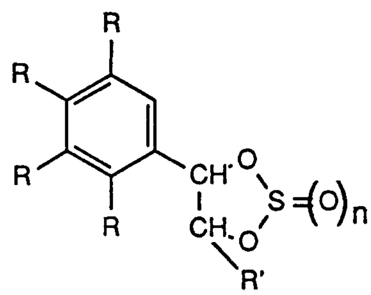

(3)

worin R und R' die angegebene Bedeutung haben und n = 1 oder 2, mit einer Aminogruppen enthaltenden Verbindung (4) unter Ringöffnung bei Temperaturen im Bereich von 50 °C bis 200 °C in Gegenwart von protischen Lösungsmitteln oder Lewis-Säuren umgesetzt,

(b) in den so erhaltenen 2-Phenyl-2-aminoethanol-Verbindungen (5) freie OH-Gruppen und NH-Gruppen allein oder jeweils zusammen durch Schutzgruppen, ausgewählt aus Alkyl-, Acyl-, Silyl- und Diacylresten geschützt und

(c) der Ringschluß unter Bildung der 1-Alkyl-1,2,3,4-tetrahydro-isochinolin-derivate (1) über eine am Stickstoff befindliche Acetaldehyd- oder Acetaldehyddiacetalgruppe bei Temperaturen im Bereich von -20 °C bis +50 °C in Gegenwart von Säuren durchgeführt wird.

**2.** Verfahren nach Anspruch 1, dadurch gekennzeichnet, daß die Ringöffnung der Verbindungen (2) oder (3) in der Verfahrensstufe (a) mit Ammoniak oder Alkylamin unter Druck durchgeführt und die so erhaltenen 2-Phenyl-2-aminoethanole oder 2-Phenyl-2-alkylaminoethanole mit Halogenacetaldehyden oder Halogenacetaldehyddialkylacetalen in Gegenwart einer Base, oder mit Ethylenoxid unter Druck und anschließender Oxidation der primären Alkoholgruppe zur Acetaldehydgruppe umgesetzt werden.

**3.** Verfahren nach Anspruch 1, dadurch gekannzeichnet, daß die Ringöffnung der Verbindungen (2) oder (3) in der Verfahrensstufe (a) mit Aminoacetaldehyddialkylacetalen oder Alkylaminoacetaldehyddialkylacetalen im Lösungsmittelgemisch Dichlormethan / iso-Propanol im Volumenverhältnis 9:1 durchgeführt wird.

**4.** Verfahren nach Anspruch 1, dadurch gekennzeichnet, daß bei der Einführung der Schutzgruppen in die Verbindungen (5) in der Verfahrensstufe (b) die OH- und NH-Gruppen entweder allein durch Umsetzung mit Alkylhalogenid, Acylhalogenid, Acylanhydrid oder jeweils zusammen durch Umsetzung mit Oxalylchlorid, Dialkylcarbonat oder N,N'-Carbonyl-diimidazol geschützt werden.

5. Verfahren nach Anspruch 1, dadurch gekennzeichnet, daß der Isochinolinringschluß in der Verfahrensstufe (c) in Gegenwart von wässrigen Mineralsäuren und mit Wasser mischbaren Lösungsmitteln im Volumenverhältnis 10 : 90 bis 90 : 10 durchgeführt wird.

6. Verfahren nach Anspruch 1, dadurch gekennzeichnet, daß der Isochinolinringschluß in der Verfahrensstufe (c) in Gegenwart von Lewis-Säuren unter wasserfreien Bedingungen durchgeführt wird.

7. Verfahren zur Herstellung von optisch aktiven Verbindungen (1), dadurch gekennzeichnet, daß in der Verfahrensstufe (a) optisch aktive Ausgangsverbindungen (2) oder (3) eingesetzt werden.

8. Verfahren zur Herstellung von erythro-Verbindungen (1), dadurch gekennzeichnet, daß in der Verfahrensstufe (a) trans-konfigurierte Ausgangsverbindungen (2) oder (3) eingesetzt werden oder daß in der Verfahrensstufe (b) threo-Verbindungen (5) durch Behandlung mit Trifluoressigsäure / Trifluoressigsäureanhydrid in die erythro-Verbindungen (5) umgewandelt werden.

9. Verfahren zur Herstellung von threo-Verbindungen (1), dadurch gekennzeichnet, daß in der Verfahrensstufe (a) cis-konfigurierte Ausgangsverbindungen (2) oder (3) eingesetzt werden oder daß in der Verfahrensstufe (b) erythro-Verbindungen (5) durch Behandlung mit Trifluoressigsäure/Trifluoressigsäureanhydrid in die threo-Verbindungen (5) umgewandelt werden.

10. Verwendung der 1-Alkyl-1,2,3,4-tetrahydro-isochinolinderivate (1) gemäß Anspruch 1 für die Herstellung von Tetrahydro-isochinolinen vom Reticulintyp oder vom Norreticulintyp durch reduktive Entfernung der OH-Gruppen oder der mit Schutzgruppen versehenen OH-Gruppen durch Reduktion mit komplexen Hydriden oder durch katalytische Hydrierung.

11. Verwendung der 1-Alkyl-1,2,3,4-tetrahydro-isochinolinderivate (1), die am Stickstoff eine Acylschutzgruppe tragen, gemäß Anspruch (1) für die Herstellung von Isochinolinen vom Papaverintyp durch mit Säuren katalysierte Hydrolyse unter Abspaltung von Acyl- und anderen Schutzgruppen und gleichzeitiger Entfernung der Hydroxylgruppen.

12. Abänderung des Verfahrens nach Anspruch 1 zur Herstellung von 1-Alkyl-1,2-dihydro-isochinolinderivaten, dadurch gekennzeichnet, daß die Ringöffnung der Verbindungen (2) oder (3) in der Verfahrensstufe (a) mit Aminoacetaldehyddialkylacetalen durchgeführt wird und die Verfahrensstufen (b) und (c) gemeinsam durchgeführt werden, derart, daß Acylierungsmittel ausgewählt aus Carbonsäurehalogeniden in Gegenwart von Basen, ausgewählt aus tert. Aminen und Pyridin unter wasserfreien Bedingungen verwendet werden.

13. Verwendung der 1-Alkyl-1,2-dihydro-isochinolinderivate gemäß Anspruch 12 für die Herstellung von Isochinolinen vom Papaverintyp durch Aromatisierung mittels Säuren oder Hydrazin.

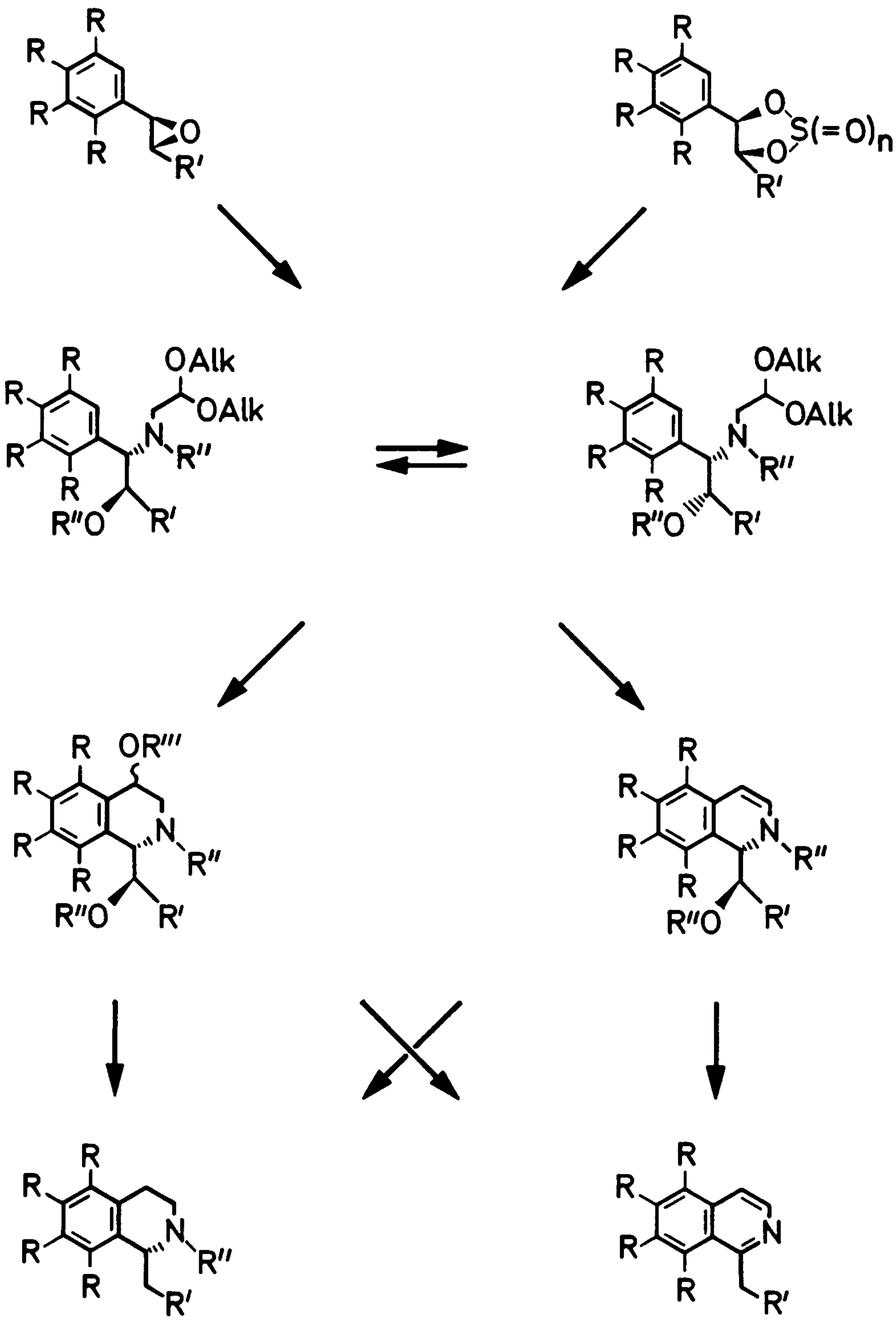

RETICULIN
NORRETICULIN
LAUDANOSIN
LAUDANOSDLIN
CARNEGIN
...ETC.

PAPAVERIN
...ETC.

Europäisches Patentamt

# EUROPÄISCHER RECHERCHENBERICHT

Nummer der Anmeldung

EP 91113377.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| A | US - A - 3 452 010 (POHLKE) * Spalte 3, Zeile 54 - Spalte 4, Zeile 59 * -- | 1 | C 07 D 217/24<br>C 07 D 217/20<br>C 07 D 491/056<br>C 07 D 498/04 |
| A | CHEMICAL ABSTRACTS, Band 108, Nr. 9, 29. Februar 1988, Columbus, Ohio, USA LENZ G. "The synthesis of the isoquinoline alkaloid calycotomine" Seite 711, Spalte 2, Zusammenfassung-Nr. 75 671b & Heterocycles, 26(3), 721-30 (1987) -- | 1 | |
| Y | EP - A2 - 0 214 905 (MITSUBISHI CHEM.) * Ansprüche 1,9 * -- | 1(C), 5,6 | |
| Y | CHEMICAL ABSTRACTS, Band 98, Nr. 9, 28. Februar 1983, Columbus, Ohio, USA YAMADA K. et al. "Studies on 1,2,3,4-tetrahydroisoquinolines" Seite 665, Spalte 2, Zusammenfassung-Nr. 72 504p & Chem. Pharm. Bull., 30(9), 3197-201 (1982) ---- | 1(C), 5,6 | **RECHERCHIERTE SACHGEBIETE (Int. Cl.⁵)**<br>C 07 D 217/00<br>C 07 D 491/00<br>C 07 D 498/00 |

Der vorliegende Recherchenbericht wurde für alle Patentansprüche erstellt.

| Recherchenort | Abschlußdatum der Recherche | Prüfer |
|---|---|---|
| WIEN | 13-11-1991 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument